# EUROPEAN PATENT APPLICATION

(11) **EP 3 124 493 A1**
(43) Date of publication of application: **01.02.2017**
(21) Application number: 15179384.1
(22) Date of filing: 31.07.2015
(51) Int. Cl.: C07K 14/415, C12N 15/82

(54) **OVEREXPRESSION OF PLANT GENES INVOLVED IN PROGRAMMED CELL DEATH FOR YIELD INCREASE**

(71) Applicant: Albert-Ludwigs-Universität Freiburg, 79085 Freiburg (DE)
(72) Inventor: Bayer, Peter, 79423 Heitersheim (DE); Yu, Qiuju, 79194 Gundelfingen (DE); Dickman, Martin, College Station, TX 77845 (US)
(74) Representative: Krauss, Jan

(57) **Abstract**

The present invention relates to a transgenic expression construct for plants, comprising an anti-apoptotic gene of the BAG family of plant genes and a promoter overexpressing said gene in a plant or part thereof. Respective transgenic plants exhibit at least one yield gain phenotype when compared to a non-transgenic plant. The present invention further relates to a method for producing a transgenic plant or part thereof, comprising introducing the transgenic expression construct of the invention into said plant or part thereof, and respective transgenic plants or parts thereof as produced.

## Description

The present invention relates to a transgenic expression construct for plants, comprising an anti-apoptotic gene of the BAG family of plant genes and a promoter overexpressing said gene in a plant or part thereof. Respective transgenic plants exhibit at least one yield gain phenotype when compared to a non-transgenic plant. The present invention further relates to a method for producing a transgenic plant or part thereof, comprising introducing the transgenic expression construct of the invention into said plant or part thereof, and respective transgenic plants or parts thereof as produced.

### Background of the invention

Yield in crops and in particular in agricultural crops is a complex multifactorial trait reflecting the interaction of the environment with all growth and development processes. Positive selection over long periods of time has led to a yield increase as a result of genomic changes that impact yield component traits like seed weight and seed number and yield related traits (for example biomass, plant architecture, adaptation and stress resistance).

By 2050, the world population is expected to reach nearly 10 billion. To sustainably provide sufficient food for this increasing population, crop productivity needs to increase by more than 40% compared to the current level. This is a challenge because there is limited potential for future expansion of arable lands whilst at the same time climate predictions suggest that a larger portion of the globe will be subject to erratic environmental conditions.

To date, strategies to increase yield in crops have utilized three major approaches, namely conventional breeding, marker assisted selection and genetic engineering. Of these, genetic engineering promises a great potential since it allows the introduction of selected genes without affecting the desirable characteristics of an elite genotype.

Genetic engineering has been used to improve the resistance of crops against environmental stresses like drought or salinity. Some success has been achieved to reduce the losses caused by these environmental stress factors, which contribute to a major part to crop yield losses worldwide.

While improving the tolerance against environmental stresses may help to reduce the crop yield losses, the solutions found today in this regard do not normally lead to a yield increase compared to the wild type in the absence of such external stress factors.

Programmed cell death (PCD) is essential for all multicellular organisms. The decision of whether a given cell should live or die is the result of a competition between anti-apoptopic and pro-apoptopic (pro-death) proteins and signal transduction pathways.

Compared to the vast molecular knowledge base available for PCD (frequently referred to as apoptosis) in animal or mammalian cells, much less data are available for plant PCD process. It remains undisputed that genetic programs leading to cellular suicide do exist in plants, being involved in resistance to disease and developmental processes. With respect to the latter, PCD pathways are known to play a role, for instance, in the male and female gametophyte development, during incompatible pollen-pistil interactions, during seed development and germination, seed coat and endosperm development, the formation of tracheary elements, aerenchyma formation and lateral and adventitious root emergence, root cap development, leaf abscission processes and senescence.

Plants often respond to pathogen attack by executing PCD programs such as the hypersensitive (HR) response. For instance, plant resistance to biotrophic pathogens are generally a result of the pathogen triggering cell death. Hemi-biotrophs and necrotrophs take over the plant HR response, thus reprogramming plant pathways, tricking the plant to undergo PCD that provides a nutritional benenfit entirely for the pathogen.

Programmed cell death in plants appears distinct from animal apoptosis; plant homologs of mammalian regulators of apoptosis are scarce using standard search algorithms. For example no sequence specific caspase homologs can be found in plant genomes; yet enzyme activities have been demonstrated. Further, there are plant proteases that mediate similar phenomena, with a similar conceptual framework but are not true caspases. Importantly, the ectopic expression of several animal anti-apoptotic genes in transgenic plants is known to confer protection from pathogens and other stresses as described by (Dickman et al., 2001; Lincoln et al., 2002). It is therefore conceivable that the divergent evolution of PCD molecular constituents has led to mechanisms in plants that may be functionally similar to animal genes and pathways independent of primary sequence. However, these cannot be easily deciphered in the absence of searchable sufficiently homologous genes.

*Arabidopsis thaliana* (At) *AtBAG4, Hsp70* and *p35* are anti-apoptotic genes that have been reported to confer tolerance to salinity and drought stresses in transgenic tobacco.

The mammalian BAG (Bcl-2-associated athanogene) family of proteins (7 homologs in humans) promote cell survival acting synergistically with Bcl-2. Various additional functions have been ascribed to BAG proteins including apoptosis, tumorigenesis, neuronal differentiation, stress response and cell cycle regulation. BAG proteins are characterized by a common C-terminally localized BAG domain mediating the interaction with Hsp70/Hsc70 molecular chaperones. Additional domains mediate the interaction with the proteasome system, the localization within cells and the interaction with transcription factors (Kabbage and Dickman, 2008).

Heat shock proteins comprise a set of powerful chaperones that are expressed in response to a variety of physiological and environmental stresses and are localized in a variety of subcellular compartments. By protein-protein interaction Hsp70 proteins can facilitate anti-apoptotic Bcl-2 proteins to inhibit apoptosis pathways at distinct key points (Joly et al., 2010). The broad-spectrum activity of Hsp70s requires the recruitment of co-chaperones and other chaperone systems (Brodsky and Bracher, 2007). Overexpression of *Hsp70* in tobacco conferred tolerance to drought stress (Cho and Hong, 2006).

The function of p35, a Baculovirus anti-apoptotic protein has been extensively studied in different organisms (Clem et al., 1991; Clem and Miller, 1994; Bump et al., 1995; Xue and Horvitz, 1995; Bertin et al., 1996). Expression of *p35* in tobacco, tomato and passion fruit significantly improved tolerance to abiotic and biotic stresses (Lincoln et al., 2002; Wang et al., 2009). Transgenic tobacco expressing *p35* displayed broad-spectrum tolerance to a range of abiotic stresses including salinity, upon further investigation this tolerance was attributed to the ability to sequester ROS (Wang et al., 2009).

A moderate low-level expression of the *AtBAG*-4 gene was shown to confer protection to plants from a variety of abiotic stresses, by inhibiting programmed cell death, while knockout plants were very susceptible to various stresses including drought tolerance, increased salt stress, increased UV-B tolerance, cold treatment and oxidant exposure. In contrast to *AtBAG1,5,7, AtBAG6* T-DNA insertion mutants are extremely susceptible to the necrotrophic fungus *B. cinerea* to which it is normally resistant, suggesting that AtBAG6 has a role in basal resistance limiting necrotrophic pathogen colonization (Kabbage and Dickman, 2008).

Hoang et al. (2015) describe the development of salinity tolerance in rice by constitutive over-expression of genses involved in the regulation of programed cell death. The authors show that the exogenous expression of anti-apoptopic genes from diverse origins, including the *AtBAG4* gene of *Arabidopsis* improves salinity tolerance in rice at the wholeplant level. However, this is related to the avoidance of losses, not to a net yield gain that surpasses standard yield potentials.

In fact, until today no way to improve yield in crops or plants in the absence of environmental stress factors and leading to an improvement in the overall harvest yield compared to the wild-type under the same condition has not been found and still remains a need to be solved.

It is therefore an object of the present invention to provide a process for the increase of yield in crops, in partcilular in the absence of environmental stresses, leading to a yield exceeding the yield in wild-type plants. Other objects of the present invention will become apparent to the person of skill when reading the following specification, examples, claims and figures of the present invention.

According to a first aspect of the present invention, this object is solved by a transgenic expression construct for plants, comprising an anti-apoptotic gene of the BAG family of plant genes comprising at least one BAG domain, or a plant homolog of said BAG gene, and a promoter overexpressing said gene in a plant or part thereof, when compared to the expression the wild-type promoter of said anti-apoptotic gene. Preferred embodiments of the present invention are set forth in the dependent claims and the detailed specification hereinafter.

It was surprisingly found that when an anti-apoptotic gene of the BAG family of plant genes was overexpressed in a plant or part thereof, a substantial increase in yield could be achieved. Their overexpression seems to delay PCD in (e.g.) endosperm cells and has additional effects leading to increased yield potential in monocot (e.g. *Oryza sativa*) and dicot (e.g.*Nicotiana tabacum*, *Arabidopsis thaliana*) plants, upon overexpression. This, for example, affects the grain size for assimilates, but also inflorescence and panicle architecture leading to increased seed numbers. In addition, constitutive expression leads to larger leaves and thicker stems and thus to an increase in overall biomass.

In the context of the present invention, the anti-apoptotic gene of the BAG family of plant genes is characterized in that it comprises at least one BAG domain as described, for example, above. BAG domains are known to functionally mediate the interaction with molecular chaperones, in particular Hsp70/Hsc70. Thus, an "anti-apoptotic gene of the BAG family" shall mean a gene, preferably a plant gene, the product of which a) promotes cell survival (i.e. is anti-apoptotic), and b) comprises a (at least one) BAG domain binding to chaperones, for example Hsp70 and/or 90. The BAG domain can be N- or C-terminally localized. The definition also includes protein complexes, wherein the proteins as involved provide at least one of these "BAG family" functions when present in said complex. Optionally, the BAG-gene can also comprise at least one Ubiquitin-like-domain (see Doukhanina et al., 2006b).

Preferred is the transgenic expression construct according to the present invention, wherein said anti-apoptotic gene is selected from a gene from *Arabidopsis thaliana* (At)- or *Oryza sativa* (Os)- or homologs thereof. Seven BAG homologs were identified in *Arabidopsis,* four of which have domain organization similar to animal members. The remaining three members contain a predicted calmodulin-binding motif near the BAG domain, a feature unique to plant Bcl-2-associated athanogene (BAG) proteins (Doukhanina et al., 2006).

The following **Table 1** shows preferred BAG proteins for use in the present invention.

| **Name/SEQ ID NO.** | **NCBI accession no** | **mass** | **length** | **Localization** |
|---|---|---|---|---|
| AtBAG1/1 | NP_200019 | 36.4 | 326 | Cytoplasm |
| AtBAG2/2 | BAB10172 | 25.5 | 232 | Microbody |
| AtBAG3/3 | NP_196339 | 34.2 | 303 | Cytoplasm |
| AtBAG4/4 | AAC14405 | 29.2 | 269 | Cytoplasm |
| AtBAG5/5 | AAC17606 | 24.5 | 215 | Mitochondrion |
| AtBAG6/6 | AAC62882 | 116.8 | 1043 | Nucleus |
| AtBAG7/7 | NP_201045 | 51.6 | 446 | Nucleus |
| OsBAG4/8 | NP_001044700.1 | | 262 | |

Other BAG genes that are useful in the present invention are found, for example, in Raphanus raphanistrum, Brassica napus, Brassica oleracea, Raphanus sativus, Brassica rapa, Triticum aestivum, Gossypium hirsutum, Vitis vinifera, Quercus robur, Nicotiana tabacum, Solanum tuberosum, Solanum lycopersicum, Solanum melongena, and Coffea canephora.

A "homolog" of the BAG gene according to the present invention shall mean a gene that encodes for a protein that exhibits at least about 60%, preferably at least about 70%, and more preferred at least about 80% or at least about 90% and even more preferred at least about 95% sequence identity to a BAG-protein as described herein, and in particular as above. Of course, this protein also has to be functional with respect to anti-apoptosis and BAG domain, as explained above.

In the context of the present invention, the term "about" shall generally mean a variability of +/- 10% of the value as indicated.

In the context of the present invention, a gene is overexpressed by a promoter, particularly in a plant or part thereof, if as a result of said expression an altered spatial distribution and/or an increased quantity of the gene product is found, compared to the expression of the wild-type (regular) promoter for this gene in, for example, a non-transgenic plant or part thereof. Preferred levels of overexpression lead to a more than 2-fold, more than 5-fold, more than 10-fold or even more than 20-fold higher expression of the gene, when compared to the expression of the wild-type (regular) promoter for this gene. Overexpression can be measured using well-known methdos in the art, such as mRNA detection (e.g. using rtPCR), promoter activity assays, and Western blotting (immunoblot) analysis.

Hoang et al. (2015) describe that exogenous expression of anti-apoptotic genes from diverse origins, *AtBAG4 (Arabidopsis)*, *Hsp70 (Citrus tristeza virus)* and *p35* (Baculovirus), significantly improved salinity tolerance in rice at the whole plant level. They demonstrated traits associated with stress-tolerant transgenic events including, improved photosynthesis, membrane integrity, ion and ROS maintenance systems, growth rate, and yield components. Anti-apoptotic genes are said to facilitate maintenance of metabolic activity at the whole plant level to create favorable conditions for cellular survival under salt stress. *AtBAG4* from *A. thaliana*, *Hsp70* from *Citrus tristeza* virus and *p35* from Baculovirus driven by the maize polyubiquitin-1 (Ubi-1) promoter were cloned into the binary vector pCAMBIA1301.

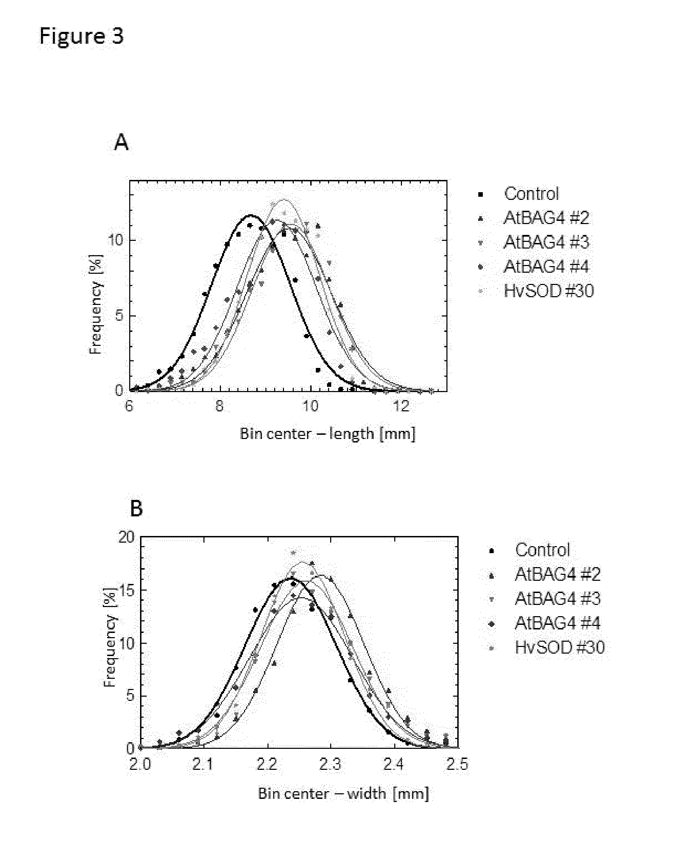

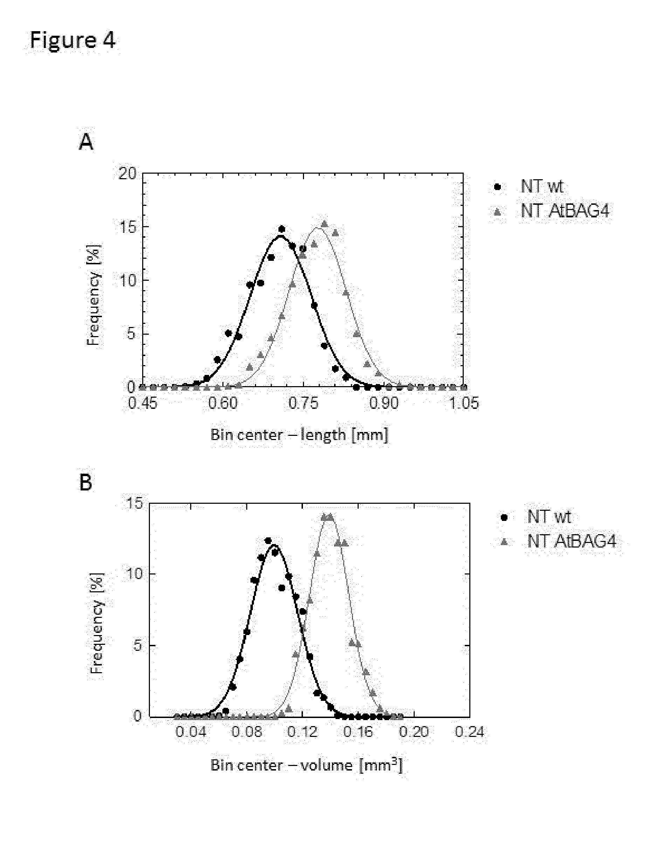

Hoang et al. (2015) disclose a yield gain only in the well-known context of protection from salinity stress. Under regular conditions, no improved effect was found when expression the gene (see suppl. data, in particular Figs. 3 and 4 at seedling and reproductive stage, respectively).

For the transgenic expression construct of the present invention, any suitable ubiquitous or tissue-specific plant promoter can be used, as long as the promoter causes an overexpression of the gene. Respective promoters are known to the person of skill, and described in the state of the art (e.g. Dutt et al., 2014). Preferred is a transgenic expression construct according to the present invention, wherein said promoter overexpressing said gene is selected from ubiquitous and tissue-specific plant promoters, such as BSV, CaMV 35S, TA29, actin, FBP7, CMPS, Lhca3.St.1, Mannopin synthase, RolD, Uep1, Ubiquitin and NapA, Gt1 (synonymously termed GluA-2), 2S, CuMFT1, Dc3, HaG3-A, LeB4, LegA, Phas, Psl, Str, and USP. For instance, to express a gene only in grains and not in leaves, a seed-specific promoter can be used. The codons of the gene can be optimized for the organism in order to overcome codon usage bias.

Yet another aspect of the present invention then relates to a method for producing a transgenic plant or part thereof, comprising introducing the transgenic expression construct according to the present invention as described above into said plant or part thereof.

Methods for making transgenic plants or parts thereof are known to the person of skill, and may involve, for example, transfer with *Agrobacterium tumefaciens*, the "gene gun" technology, electroporation or microinjection.

Consequently, another aspect of the present invention relates to a transgenic plant or part thereof, produced according to the method according to the present invention. Said transgenic plant or part thereof according to the present invention, preferably overexpresses an anti-apoptotic gene of the BAG family of plant genes comprising at least one BAG domain as described above, or a homolog of said BAG gene, such as, for example AtBAG4 or OsBAG4 as described above. Most preferred are transgenic plants comprising Gt-1 promoter-driven AtBAG4 or OsBAG4.

In general, the transgenic plant or part thereof according to the present invention can be any monocot or dicot plant, and in particular is a plant used in agriculture, e.g. a crop plant. Thus, preferably the transgenic plant is selected from *Arabidopsis,* and crop plants, such as wheat, rice, maize, barley, rye, sorghum, soy, sugar cane, pumpkin, tobacco, coffee, wine, broccoli, cauliflower, cassava, potato, and cotton.

Further preferred is the transgenic plant or part thereof according to the present invention, wherein said overexpression is non-ubiquitous (i.e. not in every part or tissue of the plant, or in substantially all parts or tissues of the plant), such as, for example, tissue-specific, e.g. specific for meristematic tissues, fast growing tissues-specific, endosperm-specific, leaf-, root-, stem-, flower-, and/or fruit-specific, or specific for certain plant cell types.

It was surprisingly found that the transgenic plants or parts thereof according to the present invention exhibit at least one yield gain phenotype, when compared to a non-transgenic plant, preferably a plant of the same variety or species without the transgene, or even without any transgene. A yield gain phenotype in the context of the present invention shall mean an increase of the mass per plant, when compared to a non-transgenic plant, also leading to an increase of mass, or grains or seeds, generated from a unit of land expressed as kilograms per hectare. According to the invention, sub-aspects of the yield gain can be selected from larger and/or longer ears or panicles, a tissue specific yield gain, a yield gain under no stress conditions, larger seeds with increased weight, seeds having an increased number of endosperm cells, and larger seed hulls having an increased number of cells.

It was furthermore found that, when employing the present invention, substantial yield gains, e.g. as defined above, could be achieved in the glasshouse. In general, yield gains of a few percentages already result in quite substantial gains. A yield gain according to the present invention is selected from more than about 5% increase in seed mass, more than about 5% increased seed weight per plant, more than about 5% increased seed number, and more than about 5% increase in overall plant biomass such as, for example, more than about 5% of biomass in rice or tobacco plants in the field. In some instances, a yield gain can be about 5%-10% increase in seed mass per plant, and about 5%-15%, preferably about 10%, increase in overall plant biomass, or even more. As can be seen in the examples below, a grain yield gain per plant of up to 60% (rice) or a yield in overall biomass (dry mass) of up to 43 % (tobacco) as found in the glasshouse can be achieved.

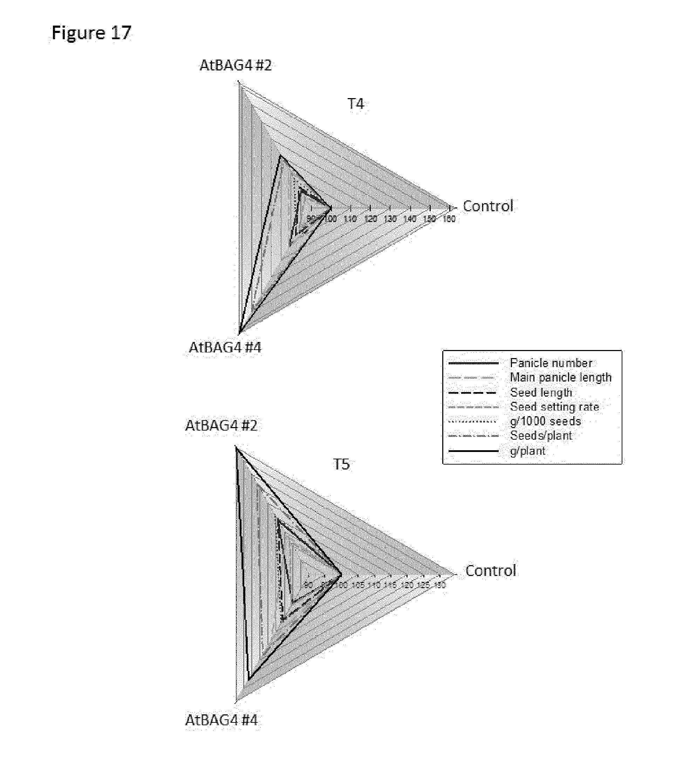

In more detail, preferred transgenic constructs, such as *AtBag4* overexpression in rice using the Gt1 promoter (endosperm- and meristematic- and fast growing tissues-specific promoter), showed 10% longer seeds (due to increased endosperm cell number, longitudinal), and 10% higher mass. The additional yield parameters such as increased panicle length accommodating an increased number of seeds per plant contribute substantially to the strongly increased grain yield noted in Figure 17. Similar results, albeit at lower levels of increase were found upon *OsBag4* overexpression in rice using the Gt1 promoter (endosperm- and meristematic- and fast growing tissues-specific promoter). *AtBag4* overexpression in tobacco using the ubiquitous 35S promoter showed about 18% increased seed weight caused by larger seeds and in the greenhouse a faster growth and the development of much larger leaves and thicker stems resulting in the dry mass increase noted above. *AtBag4* overexpression in Arabidopsis thaliana using the ubiquitous 35S promoter showed about 25% increased seed weight. These constructs thus serve as proof of concept for the overall invention, and in particular for the alternative successful embodiments (which could be combined as well) of using endosperm- and meristematic- and fast growing tissues-specific promoter or a ubiquitous promoter.

Yet another aspect of the present invention then relates to a method for identifying a yield gain plant or part thereof, comprising identifying the overexpression and/or a modification of an anti-apoptotic gene as described herein selected from genes of the BAG family of plant genes comprising at least one BAG domain, or a plant homolog of said BAG gene in said plant or part thereof, compared to a plant not overexpressing said anti-apoptotic gene and/or comprising said modification, and identifying said yield gain plant or part thereof based on said overexpression. Preferred is the method according to the present invention which further comprises identifying the underlying marker for said overexpression yield gain and/or identifying said modification, and preferably further isolating said marker.

This aspect of the present invention allows to select for and identify yet unknown favorable alleles (mutants) of genes of the BAG family of plant genes comprising at least one BAG domain, or a plant homolog of said BAG gene(s) that lead to a yield gain phenotype as described herein. The yield gain preferably is the result of an overexpression of said gene, but can also be based on functionally improved modifications, such as mutations or gene duplications of said BAG gene(s). The plants used for this method can either be obtained from spontaneously mutated plants, but the method can also comprise a mutagenesis step (for example using gene editing, such as, for example introduction of mutations by Crispr-Cas or TALEN), and then a screening for respective plants and/or markers. Consequently, another aspect of the present invention relates to a plant or part thereof as identified according to the method according to the present invention.

As above, a yield gain phenotype in the context of the present invention shall mean an increase of the mass of a plant or parts thereof, when compared to a non-transgenic plant, including but not restricted to mass of grains or seeds, leaves, stems, roots, tubers and fruit generated from a unit of land expressed as kilograms per hectare. According to the invention, sub-aspects of the yield gain can be selected from larger and/or longer ears or panicles, a tissue specific yield gain, a yield gain under no stress conditions, larger seeds with increased weight, seeds having an increased number of endosperm cells, and larger seed hulls having an increased number of cells. As above, a yield gain according to the present invention is selected from more than about 5% increase in seed mass, more than about 5% increased seed weight per plant, more than about 5% increased seed number, and more than about 5% increase in overall plant biomass such as, for example, more than about 5% of biomass in rice or tobacco plants in the field. In some instances, a yield gain can be about 5%-10% increase in seed mass per plant, and about 5%-15%, preferably about 10%, increase in overall plant biomass, or even more.

The inventors studied PCD-related processes occurring in the developing rice grain and during panicle development as a pathway that has the potential of increasing yield, when downregulated. They showed that a player of canonical PCD, the anti-apoptotic *AtBAG4* gene, delays PCD in endosperm cells and has additional effects leading to increased yield potential in monocot (*Oryza sativa*) and dicot (*Nicotiana tabacum*, *Arabidopsis thaliana*) plants, upon overexpression. This affects the grain sink size for assimilates, but also inflorescence and panicle architecture leading to increased seed numbers.

Further advantages, properties, and features of the present invention become apparent by the following description of preferred examples of the present invention with reference to the enclosed Figures, nevertheless, without being limited thereto. For the purposes of the invention, all references as cited herein are incorporated by reference in their entireties.

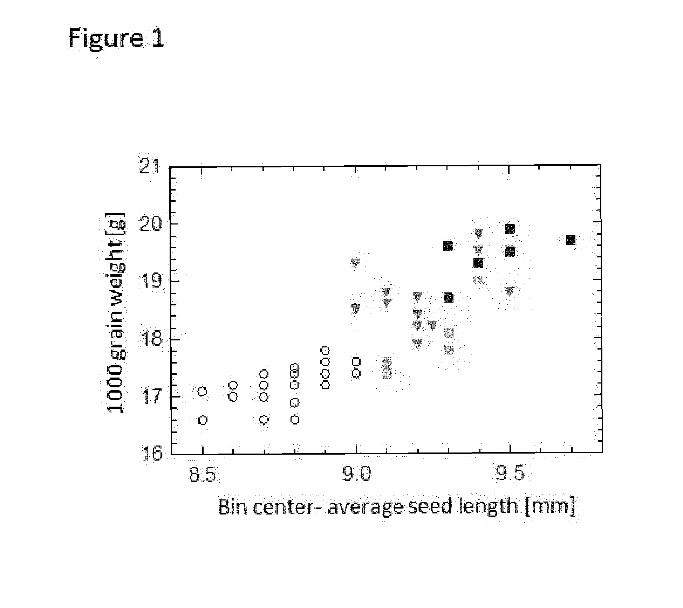

Figure 1 shows that rice seed length correlates with seed weight. Independent T2 events were preselected an analyzed in the T3 seed generation. Black circles, control; green triangles, *HvSOD* events; dark blue squares *AtBAG4* events; light blue squares, *OsBAG4* events. Individual data points represent mean values (n=100).

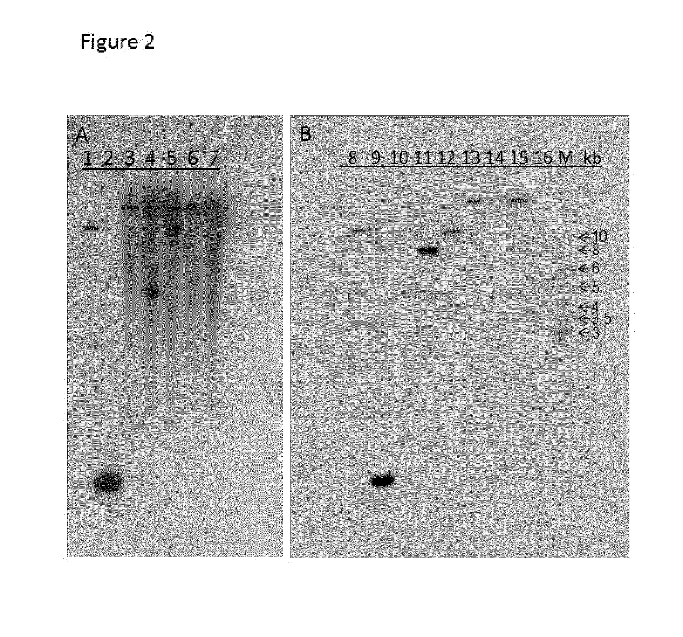

Figure 2 shows Southern blots of preselected events. Transformation events in the T2 generation were preselected by segregation analysis for single integration and *Eco*RI-digested DNA of the resulting leaf material was probed. A, *OsBAG4* events; B, *AtBAG4* events. Lanes 1,2 and 8,9 represent the linearized transformation vector and a 0.8 kb PCR amplicon, respectively, as positive controls. All other lanes represent independent transformation events, among which there are the singly integrated *AtBAG4* events s #2, #3 and #4 (lanes 11, 12, 13, respectively). Two singly integrated *OsBAG4* events are shown in A (lanes 4, 5); lane 7 and 16 represent the control, and the former also hybridizing with the internal *OsBAG 4* gene.

Figure 3 shows seed length and width distribution in selected rice events. A, length distribution of control seeds and of the T5 generation of *AtBAG4* and *HvSOD* overexpressing seeds. The event numbers are indicated. (n= > 1300 for every data set composed of randomly selected grains of 14 siblings randomly planted in the greenhouse. Events #2,3,4 *AtBAG*; event #30, *HvSOD.* The data are based on a 3D analysis using a Microtrac Dynamic Image Analyzer instrument. The curves were fitted with the Gauss equation R² = 0.94-0.97; width R² = 0.98 - 0.99.

Figure 4 shows seed metrics of Control and *35S::AtBAG4* overexpressing tobacco seeds. Seeds from 6 siblings of the control (black line) and 5 plants of *35S::AtBAG4* overexpressing events (green line) were measured using Microtrac Dynamic Image Analyzer instrument. The curves were fitted with the Gauss equation. A, seed diameter (R² = 0.98 control; R² = 0.99 *AtBAG4* events); B, seed volume (R² = 0.98 control and *AtBAG4* events). n = > 1100 seeds (mixed from the different control and overexpressing plants).

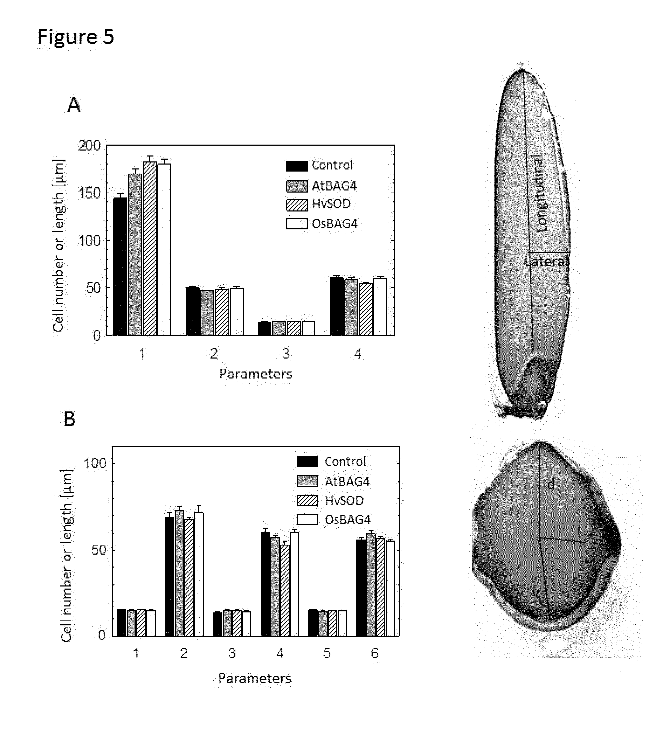

Figure 5 shows that larger seeds are caused by an increased endosperm cell number. T3 seeds of homozygous events were sectioned and morphological parameters determined along the axes indicated. A, Longitudinal sections. 1, Longitudinal cell number; 2, longitudinal cell diameter; 3, lateral cell number; lateral cell diameter. B, Cross sections; dorsal (d), ventral (v) and lateral (1) axes were defined to account for the anisodimetric shape. 1, Dorsal cell number; 2, dorsal cell diameter; 3, ventral cell number; 4, ventral cell diameter; 5, lateral cell number; 6, lateral cell diameter. Measurements were made using the ImageJ software; n = ≥ 6 per event.

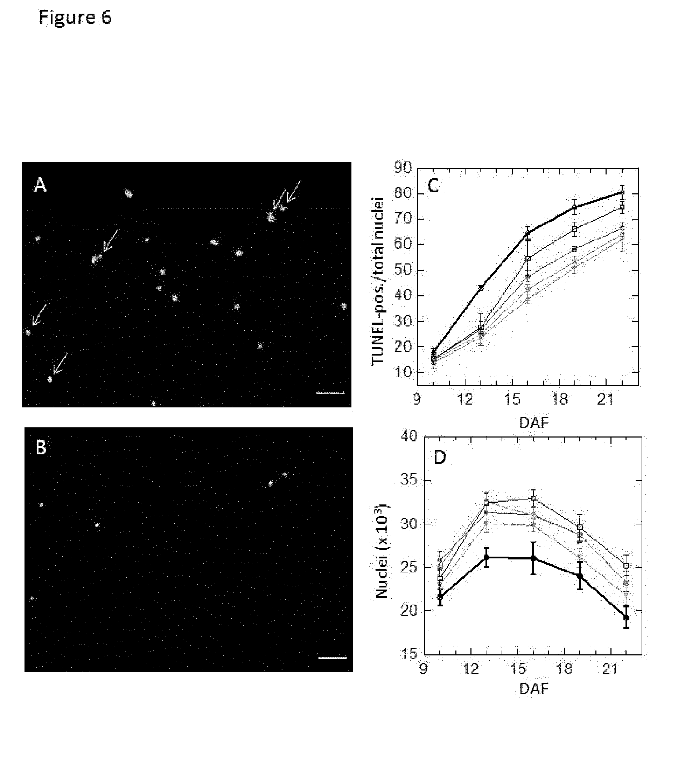

Figure 6 shows that transgenic events have a lower proportion of TUNEL-positive nuclei. Isolated rice endosperm nuclei derived from spikelets, individually tagged at the time of flowering were double stained with DAPI (A) and with TUNEL (B) allowing to assess the proportion of apoptotic nuclei. The numeric values determined are shown in C. black line, untrans-formed control; blue line, event #30 expressing *HvSOD*; green line, event # 1 expressing *At-BAG4;* orange lines events #9 and #10 expressing *OsBAG4.* In parallel aliquots, the number of nuclei was determined based on Feulgen staining. (D), number of nuclei determined independently after Feulgen staining. Each data point represents the average (± SE) of 11 replicates of 4 seeds pools. DAF, days after flowering; bar represents 10 µm.

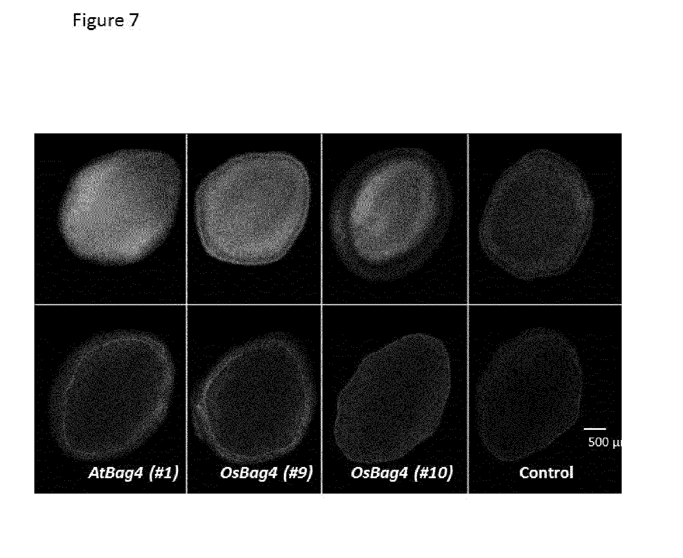

Figure 7 shows vitality stain of seed cross sections. Seeds derived from individually tagged spikelets were harvested at 16 DAF and stained with FDA (upper panel) and JC-1 (lower panel). Pictures were taken under standardized photographic conditions. Seeds expressing *At-BAG4* and *OsBAG4* showed consistently a more intense fluorescence compared to control seeds (WT).

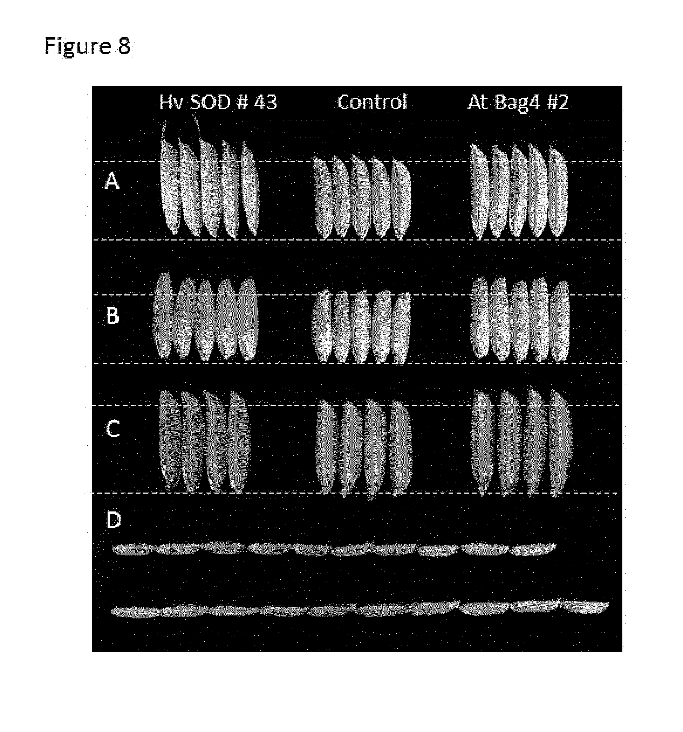

Figure 8 shows that spikelets and seed grains are equivalently increased in size. Examples of T3 seeds are shown (A) as paddy, (B) after removal of the seed coat and (C) spikelets (before fertilization). An alignment of ten corresponding spikelets is shown in (D), showing a difference of one spikelet (= 10%). The transgenic events used are indicated.

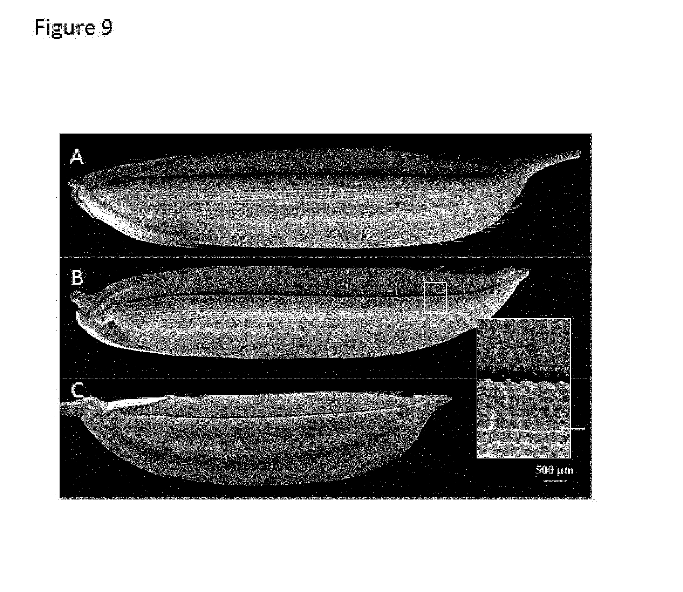

Figure 9 shows cryo-SEM of rice spikelets. Spikelets were taken at random from the middle part s of panicles and photographed. Arrow points to the 5^{th} discernible row used for cell counting. A, *HvSOD* #22; B, *OsBAG4*#33; C, Control.

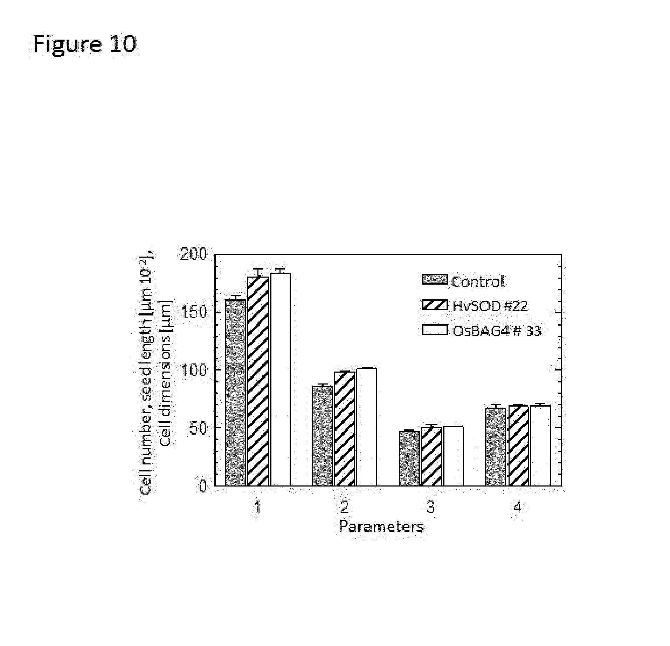

Figure 10 is based on cryo-SEM and reveals increased cell numbers in the hull. Spikelets were taken at random from the middle parts of panicles and the number and size of cells determined in the 5^{th} row of the lemma, as shown in the inset (arrow), in Figure 9. 1, Longitudinal cell number; 2, seed length; 3. cell length; 4 cell width. Data represent the mean (± SE) of three spikelets per event.

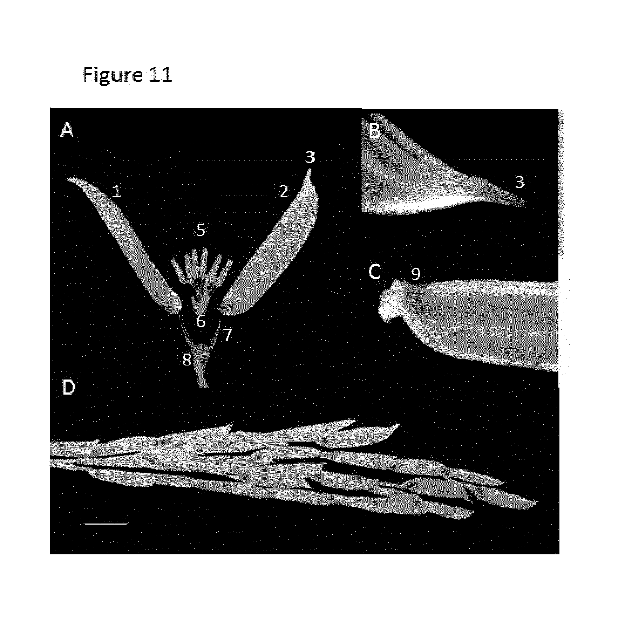

Figure 11 shows *Gtl* promoter activity in developing spikelets. The 1.85 kb version of the *Gtl* promoter used to express *HvSOD* and *BAG4* was used to express *GUS.* A discrete spatial pattern was observed in the basal growth zones of the palea (1) and lemna (2, 9) and in the outgrowth zone of the awn (3). Activity was also sometimes noted in the ovary (6). A, dissected spikelet; B, outgrowing awn; D, overview of a pre-emergent panicle. 5, stamen; 7, lodicules 8, rachilla. Bar refers to D and represents 5 mm.

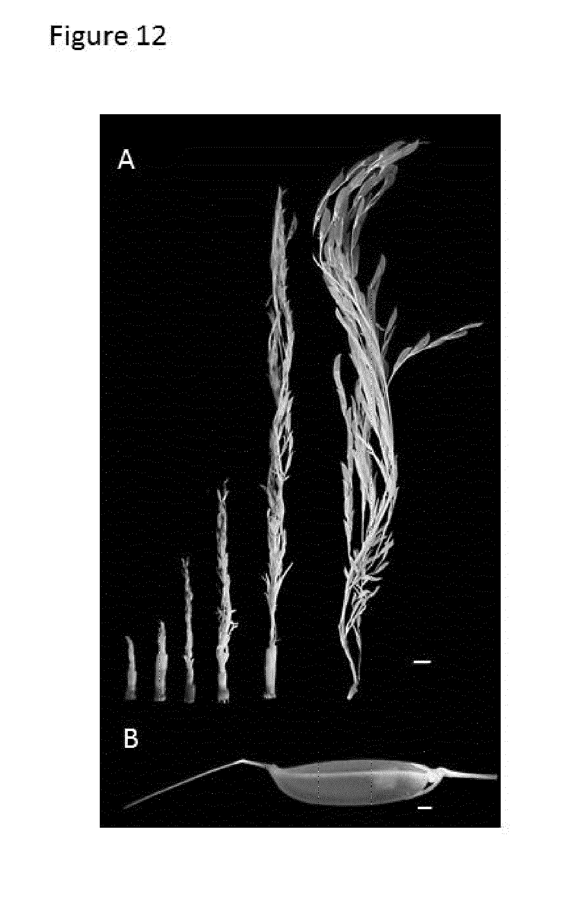

Figure 12 shows *Gtl* promoter activity in developing panicles. The 1.85 kb version of the *Gtl* promoter used to express *HvSOD* and *BAG4* was used to express *GUS.* A, six developmental stages of pre-emergent panicles. The spotted spatial expression profile shown in Figure 11 is observed from stage 3-5. When grown to almost full size (stage 6), spikelet hulls show the uniformous GUS stain, shown in B. Bars represents 5 mm (A) and 10 mm (B).

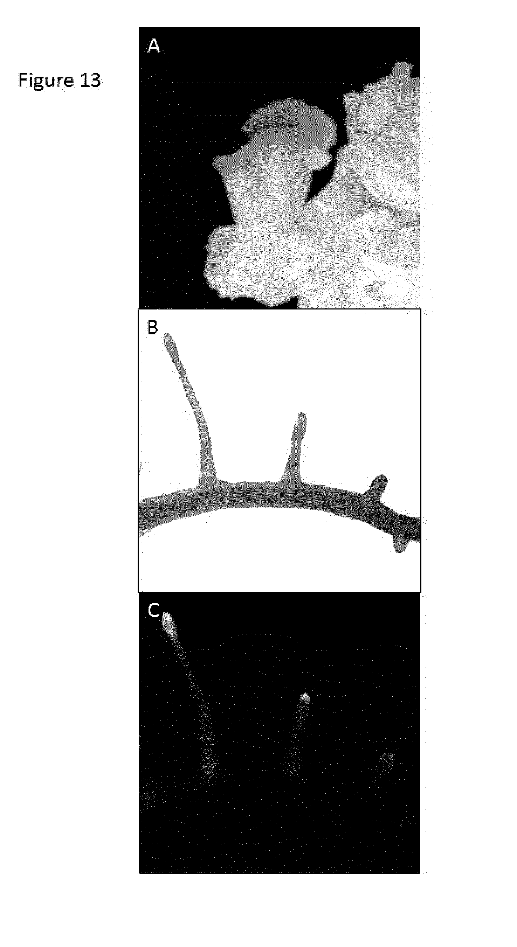

Figure 13 shows *Gtl*::reporter gene expression in dividing cells. A, GUS signal obtained in regenerating calli. B, YFP expression in root tips of regenerating plantlets.

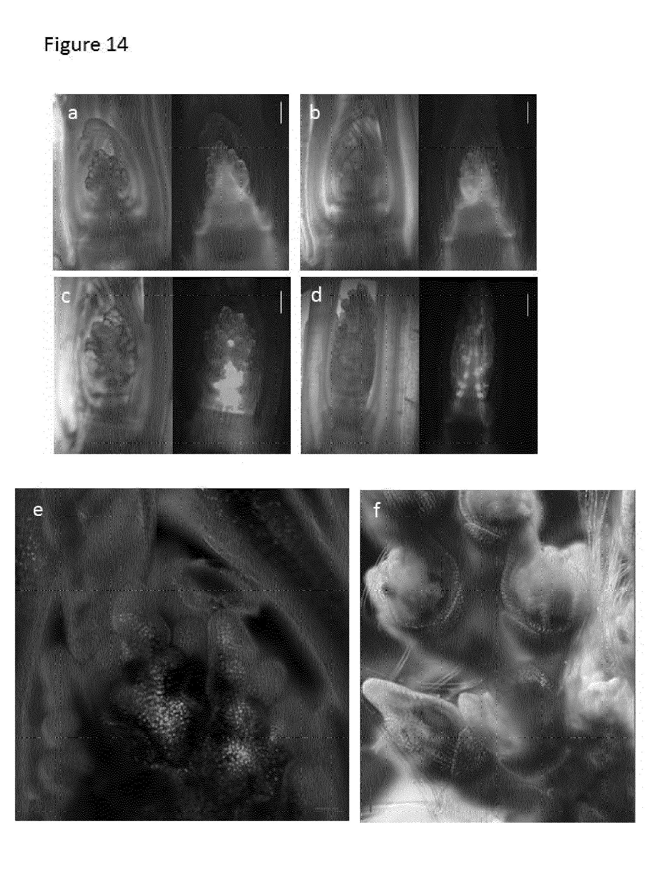

Figure 14 shows *Gtl*::YFP reporter gene expression in inflorescence primordia. Developing inflorescences are ordered according to size to reflect progressing developmental stages. Bar represents 200 µm (a-c) and 500 µm (d). The cellular expression in primordia substructures leading to panicle side branches and spikelets can be resolved by confocal microscopy (e, f; bar represents 50 µm. The nuclar localization is caused by a nuclear localization sequence translationally fused to GFP.

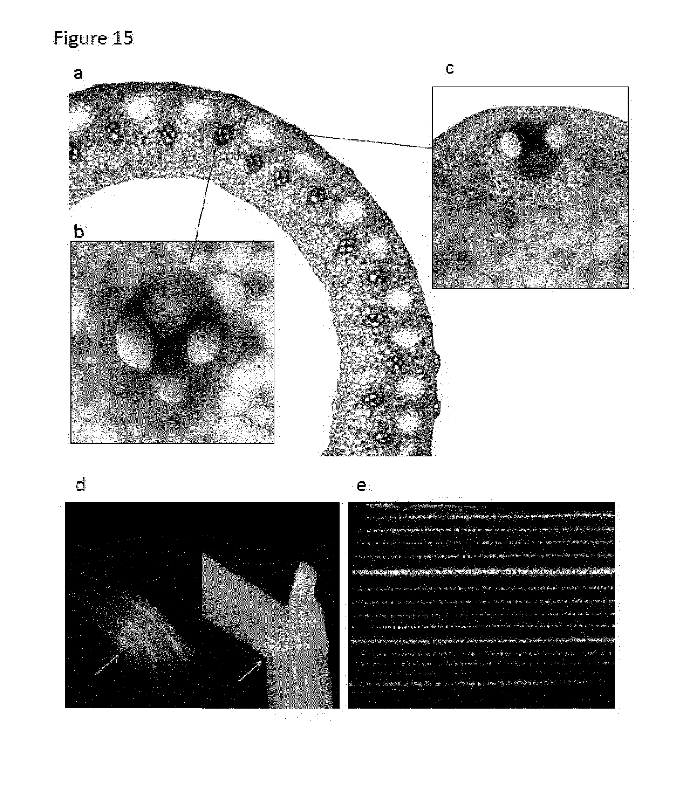

Figure 15 shows *Gtl*::reporter gene expression in vascular tissues. (a) GUS-stained, stem cross section at the 2^{nd} internode showing signals in the (b) large vascular bundles and (c), epidermal vascular tissues. (d) YFP-signals showing vascular tissue staining in the (d) collar and (e) mature leaf.

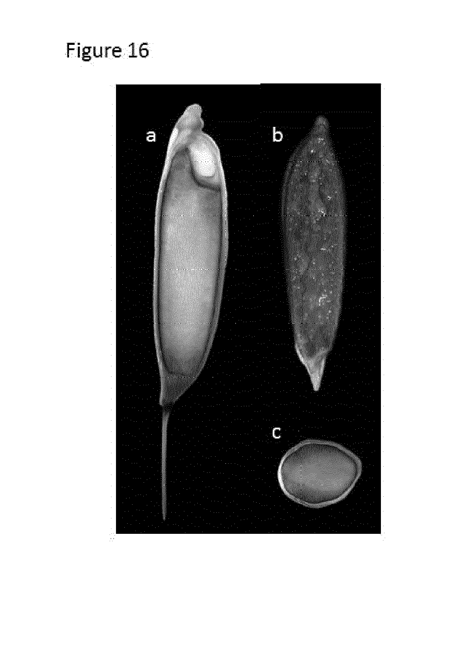

Figure 16 shows *Gtl*::reporter gene expression in rice seeds. Longitudinal (a) and transversal (c) section of a mature rice seed at 25 DAF; (b) longitudinal section of a late milky-stage seed at 10 DAF.

Figure 17 shows that several parameters contribute to increased yield per plant. Radar plot, in which the parameter changes given in Table 3 are displayed on a per cent scale (Control = 100%). All parameters displayed deviating from equilateral triangle contribute to the increased yield (gram seeds per plant; black line).

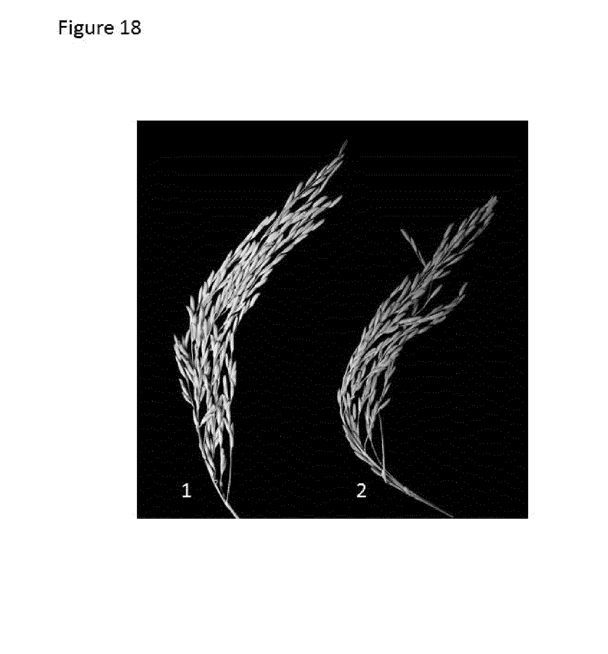

Figure 18 shows that transgenic lines show longer panicle accommodating a higher number of seeds. 1, Transgenic line overexpressing *AtBAG4* (event #2); 2, control.

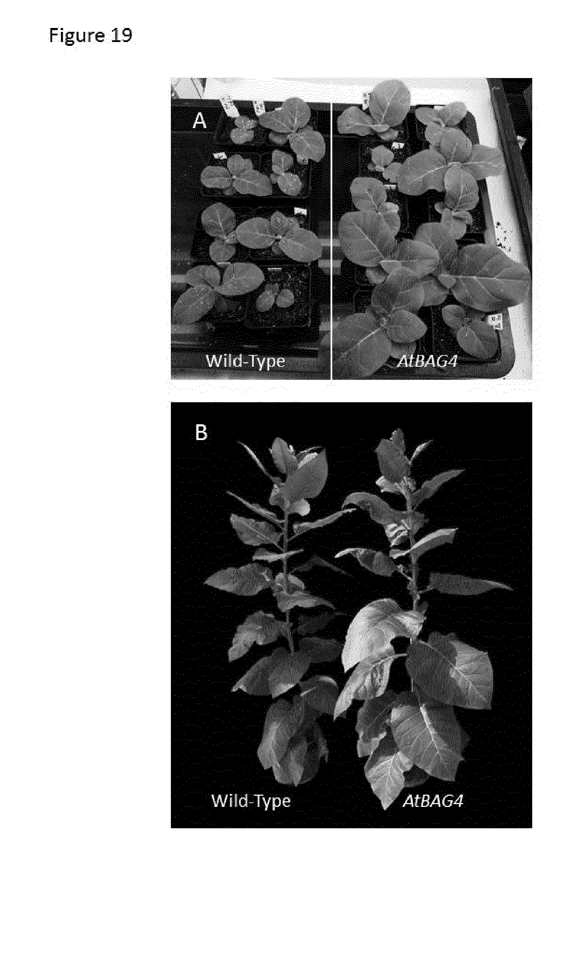

Figure 19 shows that *AtBAG4*-overexpressing plants exhibit increased leaf size in young (A) and adult (B) plants. The general leaf shape remains unchanged.

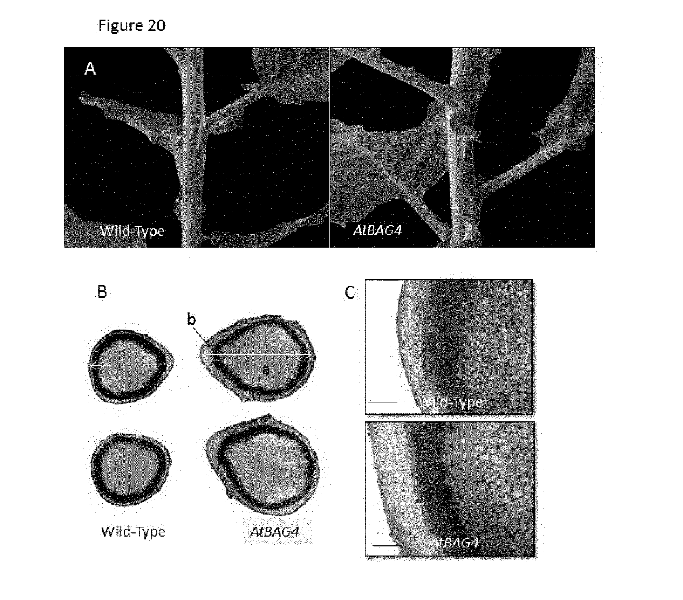

Figure 20 shows that *AtBAG4*-overexpressing plants have thicker stems. A, aspect of stems at the same reproduction scale taken at the 11^{th} leaf position. B, macroscopic view of cross section (at the 11^{th} leaf position). The white double arrow indicates the diameter and representing 16 mm (wild-type) and 22 mm (AtBAG4 overexpressor); a, pith (parenchyma); b, cortex (chlorenchyma). C, microscopic view of stem sections. Bar represents 1 mm.

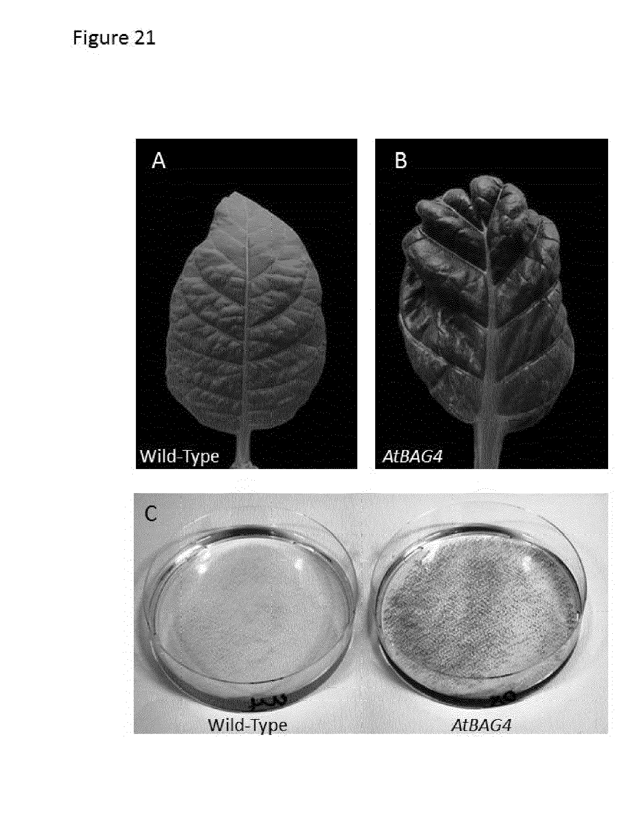

Figure 21 shows that cell proliferation is promoted by. A, young leaves of the *AtBAG4-*overexpressing plants are darker green and show a strongly proliferating phenotype characterized by the increased call mass between the leaf veins. This leads to a strongly "spinach-like" appearance which disappears when leaves grow larger. B, protoplasts were generated from in-vitro grown wild-type and *AtBAG4*-overexpressing tobacco plants Their concentration was adjusted to 2 x 10⁴ cells ml⁻¹ PNT medium and allowed to grow under a dim light/dark cycle at 27°C for seven days. The *AtBAG4* overexpressing cells show much faster cell proliferation.

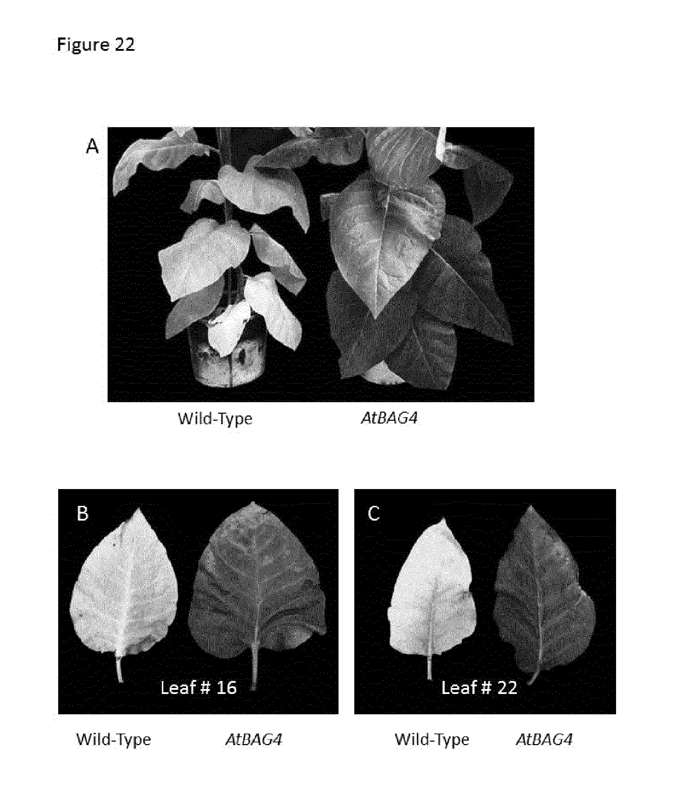

Figure 22 shows the increased longevity of *AtBAG4*-overexpressing tobacco plants. A, wild-type plants show senescence during growth, starting at the bottom at the plant, while overexpressing plants (possessing larger leaves) do not. B, delayed senescence is caused by a loss of ethylene sensing. Leaves (with no signs of senescence) at the indicated defined middle positions of plants of the same age were wrapped on the plant into plastic and exposed for 1.5 days to ethylene (generated from 2 ml of a 50 mM Ethephon solution). While wild-type leaves responded strongly, no signs of senescence were apparent in the *AtBAG4-*overexpressing leaves.

SEQ ID No. 1 to SEQ ID No. 8 show amino acid sequences of preferred *BAG-*genes (see table 1).

### Examples

*Abbreviations:* CTAB, Hexadecyl trimethyl-ammonium bromide; DAF, days after flowering; DAPI, 4',6-Diamidino-2-phenylindole; FDA, Fluorescein diacetate; JC-1,5,5',6,6'-tetrachloro-1,1',3,3'-tetraethylbenzimidazol-carbocyanine iodide; TUNEL, Terminal deoxynucleotidyl transferase-mediated dUTP nick end labeling.

### Material and methods

### Chemicals used

Phusion™ High-Fidelity DNA Polymerase was a product of Finnzymes. Nylon membranes (positively charged), DIG High Prime DNA Labeling and Detection Starter Kit II were from Roche. The following chemicals were purchased from Sigma-Aldrich: JC-1, DAPI, FDA, chemicals for Gibson cloning, cellulose from *Trichoderma viride,* alpha-amylase from *Bacillus licheniformis*, Feulgen reagent and toluidine blue.

### DNA constructs

*HvSOD* (Database Acc. No. AK248365.1) lacking its predicted 40-amino-acid transit peptide (ChloroP 1.1 software), individually or in the combination with a 5'-fusion transit peptide of Pea ribulose-1,5-bisphospate carboxylase/oxygenase small subunit (SSU), were synthesized (GenScript), respectively. Both were equipped with a 3'- *Bam*HI and a 5' *Hin*dIII site. The excised fragments were ligated into an accordingly digested pCambia1380 -derived binary vector harboring the *hptII* selectable marker gene and the 1.8 kb rice endosperm- specific glutelin *Gt1* (syn. *GluA-2)* promoter (Okita et al., 1989; Wu et al., 1998). This yielded *pC1380-Gt1-SSU-SOD* and *pC1380-Gt1-SOD.*

*AtBAG4* was amplified using Phusion™ High-Fidelity DNA Polymerase and suitable primers. The purified, *Sma*I/ *Sal*I-digested PCR product was inserted into the same binary vector. The *OsBAG4* fragment was obtained by *Bam*HI digestion from the original vector (*pYC30-OsBAG4*) and ligated into the *Bam*HI digested and dephosphorated backbone of the same binary vector. The resulting vectors, *pC1380-Gt1-AtBAG4* and *pC1380-Gt1-OsBAG4*, were used for Agrobacterium transformation.

To determine the tissue-specificity of the 1.85 kb *Gtl* promoter used, the vectors *pCA1380-Gtl-NLS-3vYFP* and *pCA1380-Gtl-NLS-GUS* were constructed using the same binary vector. The source vectors containing *NLS-3vYFP (pMU006)* and *NLS-GUS (pNK186)* were kindly provided by T. Laux (University of Freiburg). The *NLS-3vYFP* fragment was obtained by *Bam*HI/*Bgl*II digestion and ligated into the *Bam*HI digested and dephosphorated backbone of the same binary vector. The *NLS-GUS* fragment was obtained by PCR amplification using *pNK186* as template and suitable primers. The PCR product was mixed with *Bam*HI / *Spe*I digested backbone fragment and the ligation was done by Gibson cloning (Gibson et al., 2009). All vectors were verified by sequencing.

### Rice transformation and plant propagation

Embryogenic calli derived from immature embryos of an experimental Golden Rice 2 (GR 2 (Paine et al., 2005; Ye et al., 2000) event in an *Oryza sativa* var. japonica (cv Kaybonnet) background (hereafter termed "control") were used for the co-cultivation with Agrobacteria, hygromycin selection and plant regeneration as described (Ilg et al., 2010). After 3-4 rounds of hygromycin selection, resistant calli were subjected to regeneration. Plantlets were transferred to rooting medium for 2 weeks, and then to Yoshida solution (Yoshida et al., 1976) for 2 weeks at 28 °C under 16h/8h light-dark cycle, and transplanted to soil with a spacing of 15×10 cm for greenhouse growth under a 16h/8h light/dark cycle at 31°C/26 °C. A number of 45-70 independent events were generated for each construct. Preliminary selection for single integration was done by germinating the segregating T1 seed generation in the presence of hygromycin (80 mg l⁻¹). Events with a 3:1 germination ratio were carried into the T2 seed generation, which were subjected to the same selection. Those showing no segregation were assumed to be homozygous. Southern-blot analysis was done to verify the copy number of the insertion. Genomic DNA (20 µg) were digested with *Eco*RI, a unique restriction site localized adjacent to the 5' end of the *Gtl* promoter. The probes hybridizing with *OsBAG4* or *AtBAG4* were labeled, and the blotting carried out according to the manufacturer's instructions (DIG High Prime DNA Labeling and Detection Starter Kit II, Roche). The corresponding linearized binary vector plasmids (100 pg/lane) and cDNA fragments generated by PCR or restriction digestion (20 pg/lane) were used as positive controls.

Azygous seeds were isolated from the segregating T1 plant generation using a leaf assay for hygromycin sensitivity, essentially according to (Roy et al., 2012). An optimized concentration of 120 µg ml⁻¹ dissolved in water was applied to 2-3 cm leaf segments of 1-month-old transgenic and control plants and kept in Petri dishes at RT under which condition bleaching of azygous plants became apparent after 5 d. The bleached segments were tracked back to the corresponding plants.

### BAG4-expressing Arabidopsis thaliana and Nicotiana tabacum plants

The transformation events used expressing *AtBAG4* (Acc. At3g51780; NM 115037,7) under constitutive cauliflower mosaic virus 35S (*35S CaMV*) promoter control (Doukhanina et al., 2006). Tobacco plants (cv Glurk) were transformed with the vector *pEarlygate 104-AtBag4*, resulting in the expression of an N-terminal translational fusion YFP with AtBAG4. The data presented relate to a random mix of four integration events.

### Detection of Programed Cell Death

Flowers located at the 3rd to 6th position from the apex of the upper 3 primary branches on the panicle were individually tagged at their flowering date. Freshly harvested seeds at 10, 13, 16, 19 and 22 days after flowering (DAF) from at least 5 plants for each time point were manually dehulled. For the isolation of nuclei, the embryos were excised and the endosperms cut into small pieces, followed by fixation in acetic/ethanol (25 : 75) over night and then rehydrated with an ethanol/water graded series (75%, 50%, 25%, 0% ethanol) . The endosperm samples were kept in a water bath at 60°C for 10 min and, after rinsing with water, digested over night with 0,5% (w/w) cellulase (from *Trichoderma viride* (Sigma) and 1250 U ml⁻¹ of Type VII α-Amylase (from *Bacillus licheniformis*, Sigma) at 35°C in 1 ml/ 4 grains of McIlvaine's citrate-phosphate buffer at pH 5.0. The samples were macerated by gently pressing the pellet with a glass rod. After washing with 1X PBS and centrifugation, the pellets were weighted and resuspended in1 X PBS to reach a final concentration of 0.1 g ml⁻¹. A 200 µl aliquot was used for nuclei staining using the Feulgen method as described (Singh and Jenner, 1982). Another 200 µl aliquot was used first for TUNEL staining using the In Situ Cell Death Detection Kit POD (Roche Diagnostics) according to the manufacturer's instruction, followed by centrifugation and resuspension of the pellet in PBS and treatment with a 0.6 µM DAPI solution. This double staining was used to estimate the ratio of viable and apoptotic nuclei. The number of nuclei was determined using a counting chamber with an Axioskop 40 (Zeiss) microscope equipped with corresponding florescence filters. The isolation of nuclei proved to be necessary because TUNEL-detection is not possible in rice endosperm sections (Kobayashi et al., 2013).

The viability of endosperm cells was assessed in 16 DAF seeds with the viability stains fluorescein diacetate (FDA, Sigma-Aldrich) and 5,5',6,6'-tetrachloro-1,10,3,30-tetraethylbenzimidazolo carbocyanine iodide (JC-1, Sigma-Aldrich) as described by (Kobayashi et al., 2013). Fresh seeds were dehulled with forceps, and cut into halves submerged in H₂O with a razor blade. Sections were treated with 2 mg 1⁻¹ FDA in PBS at 25°C for 25 min in the dark, or 2 mg 1⁻¹ JC-1 at 37°C for 25 min in the dark, followed by 2 x washing with PBS at 37°C. Sections of samples were observed and photographed with a fluorescence microscope (Axiovert 135 TV, Zeiss).

### Seed morphology assessment

For the study of cells in the outer parenchyma layer of the hull, young spikelets section just before flowering were fixed with 2.5% glutaraldehyde in 100 mM potassium phosphate buffer, pH 7.0 and embedded in Epon. Cross-sections of 500 nm were made in the middle part using an Ultra Microtome (Ultracut E; Reichert-Jung) equipped with glass knife and stained with 0.25% toluidine blue. Endosperm cell number was analyzed using cross and longitudinal razor blade hand sections of freshly harvested 22 DAF seeds. Sections were stained with 0.25% toluidine blue and then photographed. Cells were counted using the ImageJ program.

The surface of young spikelets collected just before flowering was visualized by Cryo Scanning Electron microscopy (Cryo-SEM). The samples were mounted on the cryo holder with Tissue Tek O.C.T "glue", plunge frozen in nitrogen slush, transferred under vacuum into the preparation chamber of the cryo system (Gatan), sputtered with a 20 nm layer of gold and finally transferred into the SEM cold stage. The samples were kept stable at -150°C under high vacuum. Pictures were taken at 5kV acceleration voltage. Images were processed using Adobe Photoshop CS3 software. The hull length was estimated with Image J and the cell number in 5th row from the middle side of lemma was counted.

### Yield potential characteristics

Twenty four siblings of each event were planted in 57 x 17 x 10 cm trays (4 siblings per tray) which were randomly placed into the greenhouse and grown under the greenhouse conditions given above. Seeds of homozygous events were first germinated in Yoshida solution for 14 days (Yoshida et al, 1976), and transplanted to soil. The following traits for each plant were recorded at the mature stage: panicle number, seed number per plant, seed setting rate (% of filled grains rel. total spikelet number per panicle), 1000-grain weight and the grain yield in gram per plant. The distribution of seed dimensions was analyzed, after being dried at 37°C for 3 days using a Microtrac Dynamic Image Analyzer (DIA) and the data evaluated (n =200-2000) with the GraphpadPrism program.

### Promoter characterization

Cellular localization of the histochemical GUS signals and YFP fluorescence was investigated in the T0 generation of transgenic plants. Hand cuttings and inflorescences at progressing developmental stages were collected and photographed. YFP expression in vascular tissues was observed with samples from leaf collar, sheath, cross sections at the 2nd internode, the peduncles of panicle primary branches and the spikelet rachilla. Images were captured with an Axioskop 40 (Zeiss) microscope equipped with the suitable filters for YFP detection.

### Results

In brief, the examples show that the present invention led to larger seeds with increased weight in monocot and dicot seeds. Larger seeds are caused by an increased number of endosperm cells, an increased endosperm cell number caused by delayed programmed cell death, an increased number of (larger) seed grains at longer panicles, and larger seed hulls, caused by increased cell number, which allowed the seeds to grow larger. Constitutive expression in tobacco also led to larger leaves, thicker stems caused by more and larger cells and thus, in increased biomass.

### Larger seeds with increased weight in monocot and dicot seeds

Larger rice seeds relative to the control were visually apparent in transgenic events in the T2 rice seed generation of transformation events expressing individually the anti-apoptotic *OsBAG4* and *AtBAG4* gene. This appeared more pronounced with the latter which led the inventors to concentrate on these events. Interestingly, a similar phenotype was observed with events expressing superoxide dismutase from barley. As shown in Figure 1, increased size correlates with increased seed weight; roughly 10% longer seeds produced about 10 % higher mass.

A more detailed investigation was conducted with the T5 rice seed generation, after confirmation of single integration by Southern blotting (Figure 2) and continued phenotypic selection. This showed that seed sizes of *AtBAG4* events were normally distributed all showing increased length relative to the control of 8.7 ± 0.9 mm (Figure 3 A) to 9.4 ± 0.8 mm (event 2; ≈ +10%), 9.5 ± 0.7 (event 3; ≈+10%), 9.1 ± 0.8 (event 4; ≈ +6% ). Seed width showed some small increase not exceeding ≈ 2% which may be not significant (Figure 3 B). Only one out of several *HvSOD* events showing very similar characteristics (comp. Figure 1) was carried through the process, for scientific reasons. This is because of homology to known allergens (www.allergenoline.org) common to SODs excluding this transgene from further developments. A series of 40 transformation events expressing catalase from wheat (Acc. X94352.1) under the control of the same promoter did not show any seed phenotypic changes.

In order to assess whether the increased seed size extends to dicotyledonous plants, the inventors examined *Nicotiana tabacum* (Doukhanina et al., 2006) and *Arabidopsis thaliana* plants overexpressing *AtBAG4* under constitutive promoter control. Arabidopsis seeds were not amenable to 3D analysis because of their too small size, however, they were increased in weight by 24.5% relative to the control (Table 2). Tobacco seeds showed a 30% weight gain (Table 2) which corresponds to an average seed volume gain of ca. 40% and an average increase in length of ca. 10% (Figure 4). Furthermore, the *AtBAG4*-overpressing tobacco lines showed a grain mass per 10 capsules which was 19% increased while the number of capsules remained approximately constant.

**Table 2. Weight increase of Arabidopsis thaliana and Nicotiana tabacum seeds upon 35S::BAG4 expression**

| **Material** | **Seeds measured** | **mg/1000 (±SE)** | **mg/1000 seeds** | **Inc. weight (%)** |
|---|---|---|---|---|
| ***Arabidopsis thaliana*** | | | | |
| WT, sibling 1 | 490 | 15.3 | | |
| WT, sibling 2 | 706 | 15.0 | | |
| WT, sibling 3 | 808 | 15.7 | 15.4±0.4 | 0.0 |
| *35S::AtBAG4* #1, sibling 1 | 778 | 19.0 | | |
| *35S::AtBAG4* #1, sibling 2 | 774 | 19.6 | | |
| *35S::AtBAG4* #1, sibling 3, | 720 | 18.8 | 19.4±0.4 | 24.5 |

| ***Nicotiana tabacum*** | | | | |
|---|---|---|---|---|
| WT, sibling 1 | 321 | 81.3 | | |
| WT, sibling 2 | 547 | 81.2 | | |
| WT, sibling 3 | 523 | 99.4 | | |
| WT, sibling 4 | 786 | 84.3 | 82.2±0.9 | |
| WT, sibling 5 | 634 | 74.2 | | |
| WT, sibling 6 | 615 | 76.1 | | 0.0 |
| *35S::AtBAG4* #1 sibling 1 | 526 | 106.3 | | |
| *35S::AtBAG4* #1 sibling 2 | 515 | 104.2 | | |
| *35S::AtBAG4* #1 sibling 3 | 518 | 105.4 | | |
| *35S::AtBAG4* #1 sibling 4 | 500 | 113.1 | 107.3±0.4 | 30.5 |

### Larger seeds are caused by an increased number of endosperm cells

Randomly selected T4 seeds of successful homozygous rice events were harvested and the number of endosperm cells determined in longitudinal and transversal sections. This showed that the number of endosperm cells was increased by maximally 20% in the longitudinal direction (Figure 5 A), thus in comparable range with increased seed weight and size. No significant changes with respect to cell sizes longitudinal and lateral directions were observed. The latter also did not reveal differences in cell number; thus it can be concluded that increased seed size is predominantly determined by an increased cell number along the longitudinal axis. This is corroborated by equivalent measurements carried out with cross sections (Figure 5B), where these metrics showed no significant changes.

### Increased endosperm cell number caused by delayed programmed cell death

Using a DAPI/TUNEL double staining with isolated rice endosperm nuclei, the proportion of apoptotic nuclei containing fragmented DNA was estimated. As shown in Figure 6C, the transgenic events expressing the anti-apoptotic *AtBAG4, OSBAG4* as well as *HvSOD* all showed a diminished proportion of TUNEL-positive nuclei over the entire time course. Consequently, the total number of detectable endosperm nuclei was higher in the same transgenics as compared to the untransformed control (Figure 6 D). Viability staining of seed cross sections was used to visualize this process (Figure 7). This higher intensity of fluorescence in the transgenic endosperms indicates the presence of more viable cells.

### Larger seed hulls, caused by increased cell number, allow seeds to grow larger

The size and shape of the rice grain is determined by the spikelet hull before fertilization, which consists of the palea and lemma acting as a limiting frame within which grain filling occurs. It is long standing knowledge that, when allowed to grow without this restriction, kernels grow larger (T. Takeda, 1990). Moreover, factors affecting spikelet hull size and shape will impact seed grain dimensions, such as *OsSPL16* (GW8), encoding a member of the SBP domain family of transcription factors (Wang et al., 2012), *GW2*, encoding a RING-type ubiquitin E3 ligase (Song et al., 2007), *qSW5*, with unknown function (Shomura et al., 2008); *OsPPKL1-3*, encoding a Kelch-like repeat domain protein phosphatase (Zhang et al., 2012); *GS3,* encoding a regulatory transmembrane protein and *GS5*, a putative serine protease (Li et al., 2011).

Given this correlation, the larger seed grains observed upon *BAG4* and *SOD* expression should be preceded by the outgrowth of larger spikelets. As shown in Figure 8, this condition is met; the about 10% increased seeds size is accompanied by an equivalently increased spikelet size. To investigate the underlying cause, spikelets (of different events than shown in Figure 8) were investigated by Cryo-SEM, photographed, and the cell number determined as shown in Figure 9. The quantitative analysis across several spikelets gave the result shown in Figure 10. The increase in seed length of 12-14% (2) is mirrored by an increased cell number in the longitudinal direction of 13 - 17% (1). This implies that the cell size in this direction remains unchanged, a condition that is met (4), just like with cell sizes in cross sections that showed no significant increase. Thus, these results with hull cells are essentially the same as those obtained with endosperm cells (see Figure 5).

### Gt1 promoter leakiness explains increased spikelet size

The increased cell number present in spikelet hulls and the increased hull size are only explicable when strict endosperm specificity of the G*tl*-promoter (Okita et al., 1989; Zheng et al., 1993; Wu et al., 1998; Qu et al., 2008) is doubted. The inventors therefore used exactly the same 1.85 kb *Gtl* promoter version to drive the expression of the reporter genes NLS-GUS and NLS-YFP in the same (cv. Kaybonnet) rice background. The analysis of the resulting T0 plants revealed promoter leakiness in the developing spikelet's lemna and palea, before panicle emergence (Figure 11A) in dividing zones, the leaf basis and the outgrowing awn. It is only when the growth of spikelets is almost completed, shortly before emergence, that this spotted expression profile changes in favor of a uniform spatial expression across the hull (Figure 12).

Preferential expression actively dividing cells is also witnessed by reporter signals in regenerating embryogenic calli and in root tips (Figure 13). Moreover, a signal was obtained in stages of inflorescence development (Figure 14) during which panicle branches and spikelet primordia form. At later stages, a strong signal is obtained in the differentiating vascular bundles. In fact, expression in vascular tissues is maintained throughout vegetative growth (Figure 15. As expected for an endosperm promoter, reporter gene expression was very strong in the endosperm of developing seeds, however, decreased expression is observed at later stages (Figure 16). This starts at the center expending outwards and can most probably be attributed to PCD which shows this pattern of histological spread in the endosperm (see Figure 7; and Kobayashi et al., 2013).

### Increased number of (larger) seed grains at longer panicles

During early generations of transgenic *BAG4* and *HvSOD* events, the inventors noted that the yield, in terms of gram seeds per plant, exceeded - by far at times - the ca. 10% yield increase that is explicable by increased seed size and weight. This led the inventors to conduct more systematic studies with two selected rice events in the T3 and T4 plant generation (yielding T4 and T5 seeds, respectively) at different times of the year. T4 and T5 seeds were harvested in April and December, respectively, and therefore differed quantitatively in several parameters determined (Table 3). As shown in Figure 17, all parameters investigated, with the exception of the seed setting rate (= filled/total seeds) and seed diameter, contributed to yield increase. Their relative contribution can be variable and have different weights. Particularly, an increase in panicle size and its secondary branches, however small in percentage, can lead to the accommodation of significantly more seeds. A typical appearance is shown in Figure 18. The reason for these changes in panicle size and the concomitant formation of more spikelets probably resides in the expression of *BAG4* in the flower primordia (Figure 14).

### Table 3. Components contributing to increased rice grain yield (g/plant).

T3 plants (producing T4 seeds) and T4 plants (producing T5 seeds) were randomly grown in the greenhouse and harvested in April and December 2014, respectively. Numbers represent the mean (±SD); n= 20 plants (T3) and 24 plants (T4). Seed parameters (n > 500) were measured manually in the T3 generation and automatically with a high speed camera (Microtrac Dynamic Image Analyzer) in the T4 plant generation allowing n > 1500.

| | **Panicle Nr./plant** | **Main Panicle length [cm]** | **Seed length [mm]** | **Inc. seed length [%]** | **Seed width [mm]** | **Inc. seed width [%]** | **Seed setting rate [%]** | **g/1000 grain** | **Inc. g/1000 grains [%]** | **Mature seeds/ plant** | **Inc. mature seeds./plant [%]** | **g/plant** | **Inc. g/plant [%]** | **Transgene copy No.** |
|---|---|---|---|---|---|---|---|---|---|---|---|---|---|---|
| **Control** | 3.3±0.8 | 22.3±3.2 | 8.5±0.7 | 0.0 | 2.0±0.1 | 0.0 | 78.2±8.6 | 16.1±0.9 | 0 | 437±78 | 0 | 6.7±1.8 | 0 | 0 |
| **T4 *AtBAG4#2*** | 3.3±0.8 | 25.2±3.1 | 9.0±0.6 | 5.7 | 2.0±0.1 | 0.0 | 75.2±5.8 | 17.3±0.3 | 7.5 | 514±70 | 17.7 | 8.1±2.2 (ns) | 21.0 | 1 |
| **T4 *AtBAG4#4*** | 3.7±0.7 | 25.1±3.0 | 9.0±0.7 | 5.7 | 2.1±0.1 | 0.0 | 79.8±5.5 | 16.9±1.0 | 5.0 | 656±205 | 50.1 | 10.9±3.0 (*p*<0.01) | 63.0 | 1 |
| **Control** | 4.4±1.2 | 19.5±1.4 | 8.7±0.9 | 0.0 | 2.3±0.04 | 0.0 | 91.4 | 17.0±0.7 | 0.0 | 548±129 | 0.00 | 9.3±2.2 | 0.0 | 0 |
| **T5 *AtBAG4#2*** | 4.8±1.4 | 20.2±1.9 | 9.5±0.9 | 9.3 | 2.3±0.02 | 0.0 | 92.9 | 18.8±0.7 | 10.2 | 668±130 | 22.0 | 12.5±2.3 P<0.01 | 34.2 | 1 |
| **T5 *AtBAG4#4*** | 4.4±1.2 | 22.0±1.7 | 9.2±0.9 | 6.2 | 2.3±0.01 | 0.0 | 92.1 | 18.2±0.5 | 6.9 | 648±172 | 18.4 | 11.8±3.3 (*p*<0.05) | 26.90 | 1 |

### Increased organ size and biomass of N. tabacum plants constitutively expressing AtBAG4

Transgenic tobacco plants expressing *35S::AtBAG4-GFP* (four events) showed pronounced growth characteristics, in addition to the larger seeds noted above. Young plantlets already showed strongly increased leaf size (Figure 19A), a feature that was maintained during growth to maturity (Figure 19B). Leaf areas were determined at the given positions on the plant (Table 4) and showed to be increased by ca. 48%. The morphological appearance of these larger leaves was unchanged. The maximal length/maximal width ratio remained largely unaffected (1.33 in the wild-type, 1.36 in the transgenics, at average; n= 6, each) and so was the number of main leaf veins (16.7 in the wild-type, 17.0 in the transgenics, at average; n= 7, each, at position #15 and #16 ).

**Table 4. Leaf areas of WT and AtBAG4-overexpressing plants (n=6, each). Leaf areas were determined at the given leaf positions on the plant (numbered from the bottom).**

| **Wild-type (n=6)** | | | ***AtBA*G4-overexpressors** | | |
|---|---|---|---|---|---|
| Leaf # 15 [cm²] | Leaf # 16 [cm²] | Average (±SE) (#15, #16) | Leaf # 15 [cm²] | Leaf # 16 [cm²] | Average(±SE) (#15, #16) |
| 287.7 | 273.6 | | 500.0 | 454.7 | |
| 206.9 | 408.2 | | 384.0 | 452.1 | |
| 319.6 | 298.7 | | 379.0 | 408.7 | |
| 326.0 | 329.2 | | 578.1 | 452.3 | |
| 371.5 | 374.0 | | 529.8 | 360.5 | |
| 236.2 | 367.1 | **316.6±31.4** | 600.0 | 508.1 | **467.3±60.4** |

All transgenic plants also showed a pronounced increase in stem thickness (19.4 ±1.8 mm, diameter; n = 7) compared to the wild-type (13.7 ± 2.2 mm, diameter; n=7), thus an average increase of ca. 42%, measured at the 15^{th} leaf position of flowering plants (Figure 20A). Cross sections of the stems (Figure 20B) show that this increase is mainly due to increased growth of the pith parenchyma (a) and the cortex (b). Measurements made with microscopic thin sections as shown in Figure 20C suggest that the increase is caused by both, an increased cell number as well as an increase in cell diameter.

At a given age of the plants (shortly before flowering), the leaves were somewhat lower in number in the *AtBAG4* overexpressing plants. The latter showed 30.1±1.6 (n=7) leaves while 39±1.6 (n=7) leaves were found in the wild-type (ca. -23%). To cope with this ambiguity potentially compensating the increased leaf size, the biomass produced was determined with mature plants (possessing seed capsules) after drying to constant weight at 70°C (n = 7; WT and *AtBAG4* overexpressors, each), separated into roots and above-ground parts (seed capsules excluded). This revealed pronounced increases in biomass of the green plant material growing above ground of ca. +43% (Table 5), while the root mass increase of ca. + 8% may not be significant.

**Table 5. Dry mass of wild-type and AtBAG4-overexpressing plants (n=6, each).**

| **Wild-type** | | ***AtBAG4*-overexpressors** | |
|---|---|---|---|
| Dry mass Above ground [g] | Dry mass Root [g] | Dry mass Above ground [g] | Dry mass Root [g] |
| 59.8 | 5.8 | 91.0 | 7.0 |
| 43.1 | 4.9 | 91.8 | 6.7 |
| 73.3 | 6.5 | 87.0 | 6.2 |
| 78.0 | 8.5 | 86.8 | 5.1 |
| 58.6 | 4.2 | 89.3 | 6.4 |
| 60.1 | 4.4 | | |
| **Av: 62.1±12.3** | **Av: 5.7±1.6** | **Av: 89.1±2.6** | **Av: 6.2±0.8** |

Taken together, *At-BAG4 -* overexpressing plants show significantly larger leaves (+40%), significantly thicker stems (+ 42%). They have a reduced leaf number (ca. -23%) at a specific age which does not compensate the increased biomass produced of ca. + 43%. This is accompanied by the production of heavier and larger seeds (see above).

### Increased cell proliferation and increased longevity is at the basis of the invention

Based on the data presented, our current understanding is that *AtBAG4*, directly or indirectly, acts as a positive regulator of cell proliferation (the sum of cell division and cell expansion) under standard growth conditions, this forming the basis of the here-presented invention. This is a property that is novel in the field of Programmed Cell Death Research implying that PCD in plants constitutes a continuous process involved in cell homeostasis and morphogenesis. Even in very young leaves (where no PCD is expected), increased cell proliferation is witnessed in the *AtBAG4* overexpressing lines (Figure 21A,B) which are greener and have a curly shape signaling rapid cell proliferation which does not cope with epidermal expansion and the outgrowth of leaf veins. This situation becomes balanced when leaves develop further. To investigate this issue out of the plant context with its changing (phyto-)hormonal environment, protoplasts were generated according to standard methods and grown in a liquid medium. This showed that the *AtBAG4*-overexpressing cells grew very significantly faster as compared to the wild-type cells (Figure 21C). This indicates that cell proliferation, as provoked by *AtBAG4* is a plant-independent cellular process. Cell proliferation as the basis is also witnessed, for instance, in rice grains (more cells), in tobacco stems (more and larger cells). The larger rice panicles accommodating more seeds is attributed to *AtBAG4 -* expression in the flower primordia (comp. Figure 14) caused by the specificity of the Gt1-promoter, this leading to enhanced spikelet formation.

Increased longevity of plants and of cells is yet another component leading to increased yield potential. Phenotypically, *AtBAG4*-overexpressing tobacco plants are also characterized by their delayed senescence (Figure 22A) which can contribute to the yield potential by allowing prolonged assimilate production. This delay is caused by the ethylene insensitivity of the transgenics. When non-senescent leaves (of the same middle positions on the plants) are treated with ethylene, the trangenics showed no response (Fig. 22B,C). The mechanism, by which *AtBAG4* can attenuate ethylene sensing, is unknown.

### Literature Cited

Bertin J, Mendrysa SM, LaCount DJ, Gaur S, Krebs JF, Armstrong RC, Tomaselli KJ, Friesen PD (1996) Apoptotic suppression by baculovirus P35 involves cleavage by and inhibition of a virus-induced CED-3/ICE-like protease. J Virol 70: 6251-6259
Brodsky J.L. and Bracher A. (2007). Nucleotide exchange factors for Hsp70 molecular chaperones in: Networking of Chaperones by Co-Chaperones, ed. G. L. Blatch (New York: Landes Bioscience/Eurekah.com), 1-12.
Bump NJ, Hackett M, Hugunin M, Seshagiri S, Brady K, Chen P, Ferenz C, Franklin S, Ghayur T, Li P (1995) Inhibition of ICE family proteases by baculovirus antiapoptotic protein p35. Science 269: 1885-1888
Cho EK, Hong CB (2006) Over-expression of tobacco NtHSP70-1 contributes to drought-stress tolerance in plants. Plant Cell Rep 25: 349-358
Clem RJ, Fechheimer M, Miller LK (1991) Prevention of apoptosis by a baculovirus gene during infection of insect cells. Science 254: 1388-1390
Clem RJ, Miller LK (1994) Control of programmed cell death by the baculovirus genes p35 and iap. Mol Cell Biol 14: 5212-5222
Dickman MB, Park YK, Oltersdorf T, Li W, Clemente T, French R (2001) Abrogation of disease development in plants expressing animal antiapoptotic genes. Proc Natl Acad Sci U S A 98: 6957-6962
Doukhanina E V, Chen S, Zalm E Van Der, Godzik A, Reed J, Dickman MB (2006a) Identification and Functional Characterization of the BAG Protein Family in Arabidopsis thaliana. J Biol Chem 281: 18793-18801
Dutt M, Dhekney S a, Soriano L, Kandel R, Grosser JW (2014) Temporal and spatial control of gene expression in horticultural crops. Hortic Res 1: 14047
Gibson DG, Young L, Chuang R, Venter JC, Iii CAH, Smith HO (2009) Enzymatic assembly of DNA molecules up to several hundred kilobases. 6: 12-16
Hoang TML, Moghaddam L, Williams B, Khanna H, Dale J, Mundree SG (2015) Development of salinity tolerance in rice by constitutive-overexpression of genes involved in the regulation of programmed cell death. Front Plant Sci 6: 1-14
Ilg A, Yu Q, Schaub P, Beyer P, Al-Babili S (2010) Overexpression of the rice carotenoid cleavage dioxygenase 1 gene in Golden Rice endosperm suggests apocarotenoids as substrates in planta. Planta 232: 691-699
Joly AL, Wettstein G, Mignot G, Ghiringhelli F, Garrido C (2010) Dual role of heat shock proteins as regulators of apoptosis and innate immunity. J Innate Immun 2: 238-247
Kabbage M, Dickman MB (2008) The BAG proteins: A ubiquitous family of chaperone regulators. Cell Mol Life Sci 65: 1390-1402
Kobayashi H, Ikeda TM, Nagata K (2013) Spatial and temporal progress of programmed cell death in the developing starchy endosperm of rice. Planta 237: 1393-400
Li Y, Fan C, Xing Y, Jiang Y, Luo L, Sun L, Shao D, Xu C, Li X, Xiao J, et al (2011) Natural variation in GS5 plays an important role in regulating grain size and yield in rice. Nat Genet 43: 1266-1269
Lincoln JE, Richael C, Overduin B, Smith K, Bostock R, Gilchrist DG (2002) Expression of the antiapoptotic baculovirus p35 gene in tomato blocks programmed cell death and provides broad-spectrum resistance to disease. Proc Natl Acad Sci U S A 99: 15217-15221
Okita TW, Hwang YS, Hnilo J, Kim WT, Aryan a P, Larson R, Krishnan HB (1989) Structure and expression of the rice glutelin multigene family. J Biol Chem 264: 12573-12581
Paine JA, Shipton CA, Chaggar S, Howells RM, Kennedy MJ, Vernon G, Wright SY, Hinchliffe E, Adams JL, Silverstone AL, et al (2005) Improving the nutritional value of Golden Rice through increased pro-vitamin A content. Nat Biotechnol 23: 482-487
Qu LQ, Xing YP, Liu WX, Xu XP, Song YR (2008) Expression pattern and activity of six glutelin gene promoters in transgenic rice. J Exp Bot 59: 2417-24
Roy S, Banerjee A, Tarafdar J, Senapati BK (2012) Detection of probable marker-free transgene-positive rice plants resistant to rice tungro disease from backcross progenies of transgenic Pusa Basmati 1. J Genet 91: 213-218
Shomura A, Izawa T, Ebana K, Ebitani T, Kanegae H, Konishi S, Yano M (2008) Deletion in a gene associated with grain size increased yields during rice domestication. Nat Genet 40: 1023-1028
Singh BK, Jenner CF (1982) A modified method for the determination of cell number in wheat endosperm. Plant Sci Lett 26: 273-278
Song X-J, Huang W, Shi M, Zhu M-Z, Lin H-X (2007) A QTL for rice grain width and weight encodes a previously unknown RING-type E3 ubiquitin ligase. Nat Genet 39: 623-630
Takeda K (1990) Inheritance of grain size and its implications for rice breeding. In: Rice genetics II, Proceedings of the Second International Rice Genetics Symposium14-18 May 1990, IRRI, pp 181 - 190. ISBN 971-22-0027-2
Wang S, Wu K, Yuan Q, Liu X, Liu Z, Lin X, Zeng R, Zhu H, Dong G, Qian Q, et al (2012) Control of grain size, shape and quality by OsSPL16 in rice. Nat Genet 44: 950-954
Wang Z, Song J, Zhang Y, Yang B, Chen S (2009) Expression of baculovirus anti-apoptotic p35 gene in tobacco enhances tolerance to abiotic stress. Biotechnol Lett 31: 585-589
Wu C, Suzuki A, Washida H, Takaiwa F (1998a) The GCN4 motif in a rice glutelin gene is essential for endosperm-specific gene expression and is activated by Opaque-2 in transgenic rice plants. 14: 673-683
Wu CY, Suzuki A, Washida H, Takaiwa F (1998b) The GCN4 motif in a rice glutelin gene is essential for endosperm-specific gene expression and is activated by opaque-2 in transgenic rice plants. Plant J 14: 673-683
Xue D, Horvitz HR (1995) Inhibition of the Caenorhabditis elegans cell-death protease CED-3 by a CED-3 cleavage site in baculovirus p35 protein. Nature 377: 248-251
Ye X, Al-Babili S, Klöti A, Zhang J, Lucca P, Beyer P, Potrykus I (2000) Engineering the provitamin A (beta-carotene) biosynthetic pathway into (carotenoid-free) rice endosperm. Science 287: 303-5
Yoshida S, Forno DA, Cock JH, Gomez KA (1976). Laboratory Manual for Physiological studies of Rice. IRRI, Las Banos, Laguna. pp.83.
Zhang X, Wang J, Huang J, Lan H, Wang C, Yin C, Wu Y, Tang H, Qian Q, Li J, et al (2012) Rare allele of OsPPKL1 associated with grain length causes extra-large grain and a significant yield increase in rice. Proc Natl Acad Sci USA 109: 21534-9
Zheng Z, Kawagoe Y, Xiao S, Li Z, Okita T, HYoshidau TL, Lin A, Murai N (1993) 5' distal and proximal cis-acting regulator elements are required for developmental control of a rice seed storage protein glutelin gene. Plant J 4: 357-366

## Claims

1. A transgenic expression construct for plants, comprising an anti-apoptotic gene of the BAG family of plant genes comprising at least one BAG domain, or a plant homolog of said BAG gene, and a promoter overexpressing said gene in a plant or part thereof, when compared to the expression the wild-type promoter of said anti-apoptotic gene.

2. The transgenic expression construct according to claim 1, wherein said anti-apoptotic gene is selected from BAG1, BAG2, BAG3, BAG4, BAG5, and BAG6, in particular selected from *Arabidopsis thaliana* (At) or *Oryza sativa* (Os).

3. The transgenic expression construct according to claim 1 or 2, wherein said promoter overexpressing said gene is selected from ubiquitous and tissue-specific plant promoters, such as BSV, CaMV 35S, CMPS, Lhca3.St.1, Mannopin synthase, RolD, Uep1, Ubiquitin and Na-pA, Gt1, 2S, CuMFT1, Dc3, HaG3-A, LeB4, LegA, Phas, Psl, Str, and USP.

4. A method for producing a transgenic plant or part thereof, comprising introducing the transgenic expression construct according to any of claims 1 to 3 into said plant or part thereof

5. A transgenic plant or part thereof, produced according to the method according to claim 4.

6. The transgenic plant or part thereof according to claim 5, wherein said transgenic plant or part thereof is overexpressing an anti-apoptotic gene of the BAG family of plant genes comprising at least one BAG domain, or a homolog of said BAG gene, such as, for example AtBAG4 or OsBAG4.

7. The transgenic plant or part thereof according to claim 5 or 6, wherein said overexpression is non-ubiquitous, such as, for example, tissue-specific, e.g. specific for meristematic tissues, endosperm-specific, leaf-, root-, stem-, flower-, and/or fruit-specific, or specific for certain plant cell types.

8. The transgenic plant or part thereof according to any one of claims 5 to 7, wherein said transgenic plant or part thereof exhibits at least one yield gain phenotype when compared to a non-transgenic plant.

9. The transgenic plant or part thereof according to any one of claims 5 to 8, wherein said transgenic plant is selected from monocot or dicot plants, such as *Arabidopsis,* and crop plants, such as wheat, rice, maize, barley, rye, sorghum, soy, sugar cane, pumpkin, tobacco, coffee, wine, broccoli, cauliflower, cassava, potato, and cotton.

10. The transgenic plant or part thereof according claim 9, wherein said yield gain is selected from more than about 5% increase in seed mass, more than 5% increased seed weight per plant, more than 5% increase in seed number, and/ or about more than 5% increase in overall plant biomass such as, for example, more than about 5% biomass of tobacco plants.

11. A method for identifying a yield gain plant or part thereof, comprising identifying the overexpression and/or a modification of an anti-apoptotic gene selected from genes of the BAG family of plant genes comprising at least one BAG domain, or a plant homolog of said BAG gene in said plant or part thereof, compared to a plant not overexpressing said anti-apoptotic gene and/or comprising said modification, and identifying said yield gain plant or part thereof based on said overexpression.

12. The method according to claim 11, further comprising identifying the underlying marker for said overexpression yield gain and/or identifying said modification, and preferably further isolating said marker.

13. The method according to claim 11 or 12, wherein said yield gain is selected from increased overall biomass, larger and/or longer ears or panicles, a tissue specific yield gain, a yield gain under no stress conditions, larger seeds with increased weight, seeds having an increased number of endosperm cells, and larger seed hulls having an increased number of cells, when compared to a plant not overexpressing said anti-apoptotic gene.

14. The method according to any one of claims 11 to 13, wherein said yield gain is selected from more than about 5%-10% increase in seed mass, and about 5%-10%, preferably about 15%, increase in overall plant biomass.

## Amended claims

### Amended claims in accordance with Rule 137(2) EPC.

1. A transgenic expression construct for plants, comprising an anti-apoptotic gene of the BAG family of plant genes is **characterized in that** it comprises at least one BAG domain having at least about 70% sequence identity to the anti-apoptotic gene BAG4 from *Arabidopsis thaliana* (At), and a promoter overexpressing said gene in a plant or part thereof, when compared to the expression the wild-type promoter of said anti-apoptotic gene.

2. The transgenic expression construct according to claim 1, wherein said anti-apoptotic gene is selected from BAG1, BAG2, BAG3, BAG4, BAG5, and BAG6, in particular selected from *Arabidopsis thaliana* (At) or *Oryza sativa* (Os).

3. The transgenic expression construct according to claim 1 or 2, wherein said promoter overexpressing said gene is selected from ubiquitous and tissue-specific plant promoters, such as BSV, CaMV 35S, CMPS, Lhca3.St.1, Mannopin synthase, RolD, Uep1, Ubiquitin and NapA, Gt1, 2S, CuMFT1, Dc3, HaG3-A, LeB4, LegA, Phas, Psl, Str, and USP.

4. A method for producing a transgenic plant or part thereof, comprising introducing the transgenic expression construct according to any of claims 1 to 3 into said plant or part there-of.

5. A transgenic plant or part thereof, produced according to the method according to claim 4.

6. The transgenic plant or part thereof according to claim 5, wherein said transgenic plant or part thereof is overexpressing AtBAG4 or OsBAG4.

7. The transgenic plant or part thereof according to claim 5 or 6, wherein said overexpression is non-ubiquitous, such as, for example, tissue-specific, e.g. specific for meristematic tissues, endosperm-specific, leaf-, root-, stem-, flower-, and/or fruit-specific, or specific for certain plant cell types.

8. The transgenic plant or part thereof according to any one of claims 5 to 7, wherein said transgenic plant or part thereof exhibits at least one yield gain phenotype when compared to a non-transgenic plant.

9. The transgenic plant or part thereof according to any one of claims 5 to 8, wherein said transgenic plant is selected from monocot or dicot plants, such as *Arabidopsis,* and crop plants, such as wheat, rice, maize, barley, rye, sorghum, soy, sugar cane, pumpkin, tobacco, coffee, wine, broccoli, cauliflower, cassava, potato, and cotton.

10. The transgenic plant or part thereof according claim 9, wherein said yield gain is selected from more than about 5% increase in seed mass, more than 5% increased seed weight per plant, more than 5% increase in seed number, and/ or about more than 5% increase in overall plant biomass such as, for example, more than about 5% biomass of tobacco plants.

11. A method for identifying a yield gain plant or part thereof, comprising identifying the overexpression and/or a modification of an anti-apoptotic gene selected from genes of the BAG family of plant genes comprising at least one BAG domain having at least about 70% sequence identity to the anti-apoptotic gene BAG4 from *Arabidopsis thaliana* (At), or a plant homolog of said BAG gene in said plant or part thereof, compared to a plant not overexpressing said anti-apoptotic gene and/or comprising said modification, and identifying said yield gain plant or part thereof based on said overexpression.

12. The method according to claim 11, further comprising identifying the underlying marker for said overexpression yield gain and/or identifying said modification, and preferably further isolating said marker.

13. The method according to claim 11 or 12, wherein said yield gain is selected from increased overall biomass, larger and/or longer ears or panicles, a tissue specific yield gain, a yield gain under no stress conditions, larger seeds with increased weight, seeds having an increased number of endosperm cells, and larger seed hulls having an increased number of cells, when compared to a plant not overexpressing said anti-apoptotic gene.

14. The method according to any one of claims 11 to 13, wherein said yield gain is selected from more than about 5%-10% increase in seed mass, and about 5%-10%, preferably about 15%, increase in overall plant biomass.


SEQUENCE LISTING

<110>  Universität Freiburg

<120>  Overexpression of plant genes involved in programmed cell death for
yield increase

<130>  U30657EP

<160>  8

<170>  PatentIn version 3.5

<210>  1
<211>  342
<212>  PRT
<213>  Arabidopsis thaliana

<400>  1

Met Met Lys Met Met Arg Asn Lys Pro Thr Asn Leu Pro Thr Ala Gly
1               5                   10                  15

Met Thr Asn Gly Gly Arg Gly Ser Gly Gly Gly Gly Gly Gly Gly Gly
            20                  25                  30

Arg Glu Ser Gly Gly Arg Asp Leu Glu Ile Arg Pro Gly Gly Met Leu
        35                  40                  45

Val Gln Lys Arg Asn Pro Asp Leu Asp Pro Val Gly Pro Pro Pro Pro
    50                  55                  60

Pro Met Ile Arg Val Arg Ile Lys Tyr Gly Ala Val Tyr His Glu Ile
65                  70                  75                  80

Asn Ile Ser Pro Gln Ala Ser Phe Gly Glu Leu Lys Lys Met Leu Thr
                85                  90                  95

Gly Pro Thr Gly Ile His His Gln Asp Gln Lys Leu Met Tyr Lys Asp
            100                 105                 110

Lys Glu Arg Asp Ser Lys Ala Phe Leu Asp Val Ser Gly Val Lys Asp
        115                 120                 125

Lys Ser Lys Met Val Leu Ile Glu Asp Pro Leu Ser Gln Glu Lys Arg
    130                 135                 140

Phe Leu Glu Met Arg Lys Ile Ala Lys Thr Glu Lys Ala Ser Lys Ala
145                 150                 155                 160

Ile Ser Asp Ile Ser Leu Glu Val Asp Arg Leu Gly Gly Arg Val Ser
                165                 170                 175

Ala Phe Glu Met Val Thr Lys Lys Gly Gly Lys Ile Ala Glu Lys Asp
        180                 185                 190

Leu Val Thr Val Ile Glu Leu Leu Met Asn Glu Leu Ile Lys Leu Asp
    195                 200                 205

Ala Ile Val Ala Glu Gly Asp Val Lys Leu Gln Arg Lys Met Gln Val
    210                 215                 220

Lys Arg Val Gln Asn Tyr Val Glu Thr Leu Asp Ala Leu Lys Val Lys
225                 230                 235                 240

Asn Ser Met Ala Asn Gly Gln Gln Lys Gln Ser Ser Thr Ala Gln Arg
                245                 250                 255

Leu Ala Pro Ile Gln Glu His Asn Asn Glu Glu Arg Gln Glu Gln Lys
            260                 265                 270

Pro Ile Gln Ser Leu Met Asp Met Pro Ile Gln Tyr Lys Glu Lys Lys
        275                 280                 285

Gln Glu Ile Glu Glu Glu Pro Arg Asn Ser Gly Glu Gly Pro Phe Val
    290                 295                 300

Leu Asp Ser Ser Ala Lys Trp Glu Thr Phe Asp His His Pro Val Thr
305                 310                 315                 320

Pro Leu Ser Ser Thr Thr Ala Lys Asn Asn Ala Ile Pro Pro Arg Phe
                325                 330                 335

Asn Trp Glu Phe Phe Asp
            340

<210>  2
<211>  302
<212>  PRT
<213>  Arabidopsis thaliana

<400>  2

Met Met Lys Met Ser Ile Gly Thr Thr Thr Ser Gly Asp Gly Glu Met
1               5                   10                  15

Glu Leu Arg Pro Gly Gly Met Val Val Gln Lys Arg Thr Asp His Ser
            20                  25                  30

Ser Ser Val Pro Arg Gly Ile Arg Val Arg Val Lys Tyr Gly Ser Val
        35                  40                  45

His His Glu Ile Ser Ile Asn Ser Gln Ser Thr Phe Gly Lys Arg His
    50                  55                  60

Lys Thr Phe Arg Val Ile Gln Tyr Phe Leu Tyr Thr Ile Arg Glu Leu
65                  70                  75                  80

Lys Lys Ile Leu Ser Gly Ala Thr Gly Val His His Gln Asp Met Gln
                85                  90                  95

Ile Ile Tyr Lys Asp Lys Glu Arg Asp Ser Lys Met Phe Leu Asp Leu
            100                 105                 110

Ser Gly Val Lys Asp Arg Ser Lys Leu Ile Leu Ile Glu Asp Pro Ile
        115                 120                 125

Ser Gln Glu Lys Arg Leu Leu Glu Leu Arg Lys Ile Ala Thr Lys Glu
    130                 135                 140

Lys Ser Ser Lys Ala Ile Ser Asp Ile Ser Phe Gln Val Glu Arg Leu
145                 150                 155                 160

Ala Gly Gln Leu Ser Ala Phe Asp Thr Val Ile Gly Lys Gly Gly Lys
                165                 170                 175

Val Glu Glu Lys Asn Leu Glu Asn Leu Met Glu Met Leu Met Asn Gln
            180                 185                 190

Leu Val Lys Leu Asp Ala Ile Ser Gly Asp Gly Asp Val Lys Leu Lys
        195                 200                 205

Lys Lys Met Gln Glu Glu Arg Leu His Lys Tyr Val Glu Ala Leu Asp
    210                 215                 220

Leu Leu Lys Ile Lys Asn Ser Arg Gln Pro Gln Thr Lys Pro Lys Pro
225                 230                 235                 240

Gln Tyr Lys Glu Arg Glu Met Leu Thr Phe Tyr Glu Glu Ala Ser Arg
                245                 250                 255

Lys Pro Thr Ala Ser Ser Ser Ser Pro Pro Val Ile Ile Thr Thr Arg
            260                 265                 270

Trp Glu Thr Phe Asp Ser Ser Ser Ala Ser Thr Ala Thr Leu Gln Pro
        275                 280                 285

Val Arg Pro Val His Pro Lys Phe Lys Trp Glu Leu Phe Asn
    290                 295                 300

<210>  3
<211>  303
<212>  PRT
<213>  Arabidopsis thaliana

<400>  3

Met Met Lys Met Asn Thr Gly Thr Ser Pro Ser Val Ile Gly Gly Gly
1               5                   10                  15

Thr Ser Gly Asn Glu Trp Glu Ser Arg Pro Gly Gly Met Val Val Gln
            20                  25                  30

Arg Arg Thr Asp Gln Asn Ser Asp Val Pro Arg Val Phe Arg Val Arg
        35                  40                  45

Val Lys Tyr Gly Ser Val Tyr His Glu Ile Asn Ile Asn Ser Gln Ser
    50                  55                  60

Ser Phe Gly Glu Leu Lys Lys Met Leu Ser Asp Gln Val Gly Leu His
65                  70                  75                  80

His Glu Asp Met Lys Val Leu Tyr Lys Asp Lys Glu Arg Asp Ser Lys
                85                  90                  95

Met Phe Leu Asp Leu Cys Gly Val Lys Asp Arg Ser Lys Leu Val Val
            100                 105                 110

Lys Glu Asp Pro Ile Ser Gln Glu Lys Arg Leu Leu Ala Lys Arg Lys
        115                 120                 125

Asn Ala Ala Ile Glu Lys Ala Ser Lys Ser Ile Ser Asp Ile Ser Phe
    130                 135                 140

Glu Val Asp Arg Leu Ala Gly Gln Val Ser Ala Phe Glu Thr Val Ile
145                 150                 155                 160

Asn Lys Gly Gly Lys Val Glu Glu Lys Ser Leu Val Asn Leu Ile Glu
                165                 170                 175

Met Leu Met Asn Gln Leu Leu Arg Leu Asp Ala Ile Ile Ala Asp Gly
            180                 185                 190

Asp Val Lys Leu Met Arg Lys Met Gln Val Gln Arg Val Gln Lys Tyr
        195                 200                 205

Val Glu Ala Leu Asp Leu Leu Lys Val Lys Asn Ser Ala Lys Lys Val
    210                 215                 220

Glu Val Asn Lys Ser Val Arg His Lys Pro Gln Thr Gln Thr Arg Phe
225                 230                 235                 240

Glu Gln Arg Asp Leu Leu Ser Phe Val Glu Glu Glu Glu Glu Glu Pro
                245                 250                 255

Arg Asn Ser Asn Ala Ser Ser Ser Ser Gly Thr Pro Ala Val Val Ala
            260                 265                 270

Ser Lys Trp Glu Met Phe Asp Ser Ala Ser Thr Ala Lys Ala Ala Glu
        275                 280                 285

Thr Val Lys Pro Val Pro Pro Arg Phe Lys Trp Glu Phe Phe Asp
    290                 295                 300

<210>  4
<211>  269
<212>  PRT
<213>  Arabidopsis thaliana

<400>  4

Met Met His Asn Ser Thr Glu Glu Ser Glu Trp Glu Val Arg Pro Gly
1               5                   10                  15

Gly Met Leu Val Gln Arg Arg Asp Asp Ala Ala Ser Ser Asp His Lys
            20                  25                  30

Pro Leu Gln Asp Pro Asp Ser Ala Ser Ala Ala Phe Ala Gln Thr Ile
        35                  40                  45

Arg Ile Thr Val Ser His Gly Ser Ser His His Asp Leu His Ile Ser
    50                  55                  60

Ala His Ala Thr Phe Gly Asp Val Lys Lys Ala Leu Val Gln Lys Thr
65                  70                  75                  80

Gly Leu Glu Ala Ser Glu Leu Lys Ile Leu Phe Arg Gly Val Glu Arg
                85                  90                  95

Asp Asp Ala Glu Gln Leu Gln Ala Ala Gly Val Lys Asp Ala Ser Lys
            100                 105                 110

Leu Val Val Val Val Glu Asp Thr Asn Lys Arg Val Glu Gln Gln Pro
        115                 120                 125

Pro Val Val Thr Lys Glu Met Glu Lys Ala Ile Ala Ala Val Asn Ala
    130                 135                 140

Val Thr Gly Glu Val Asp Lys Leu Ser Asp Arg Val Val Ala Leu Glu
145                 150                 155                 160

Val Ala Val Asn Gly Gly Thr Gln Val Ala Val Arg Glu Phe Asp Met
                165                 170                 175

Ala Ala Glu Leu Leu Met Arg Gln Leu Leu Lys Leu Asp Gly Ile Glu
            180                 185                 190

Ala Glu Gly Asp Ala Lys Val Gln Arg Lys Ala Glu Val Arg Arg Ile
        195                 200                 205

Gln Asn Leu Gln Glu Ala Val Asp Lys Leu Lys Ala Arg Cys Ser Asn
    210                 215                 220

Pro Phe Val Asp Gln Ser Lys Ala Ala Ala Val Ser Thr Glu Trp Glu
225                 230                 235                 240

Ser Phe Gly Asn Gly Val Gly Ser Leu Asn Pro Pro Pro Pro Ala Ser
                245                 250                 255

Pro Ser Ala Asn Val Thr Gln Asp Trp Glu Lys Phe Asp
            260                 265

<210>  5
<211>  215
<212>  PRT
<213>  Arabidopsis thaliana

<400>  5

Met Lys Arg Ser Arg Lys Phe Ser Ser Ser Thr Thr Thr Thr Thr Val
1               5                   10                  15

Ile His Thr Phe Tyr Asn Asp His Thr Thr Pro Pro Ala Thr Lys Glu
            20                  25                  30

Ile Pro Ile Glu Thr Pro Leu Pro Ala Thr Lys Ala Asn Val Lys Thr
        35                  40                  45

Asn Ala Thr Ala Ala Ala Ala Arg Ile Gln Ser Gly Tyr Arg Ser Tyr
    50                  55                  60

Arg Ile Arg Asn Leu Tyr Lys Lys Ile Ser Ser Ile Asn Arg Glu Ala
65                  70                  75                  80

Asn Arg Val Gln Ser Ile Ile Gln Arg Gln Glu Thr Val Asp Ala Ile
                85                  90                  95

Arg Ser Asp Glu Lys Glu Arg Leu Arg Met Asn Glu Thr Leu Met Ala
            100                 105                 110

Leu Leu Leu Lys Leu Asp Ser Val Pro Gly Leu Asp Pro Thr Ile Arg
        115                 120                 125

Glu Ala Arg Arg Lys Val Ser Arg Lys Ile Val Gly Met Gln Glu Ile
    130                 135                 140

Leu Asp Ser Ile Ser Glu Thr Lys Asp Glu Ile Gln Trp Trp Asn Tyr
145                 150                 155                 160

Asn Asp Leu Gly Gly Val Asp Ser Gly Gln Gly Gly Gly Ala Trp Pro
                165                 170                 175

Leu Tyr Trp Glu Glu Ala Val Glu Glu Glu Met Cys Arg Glu Arg Gly
            180                 185                 190

Gly Glu Glu Met Glu Arg Phe Cys Ala Gln Tyr Leu Gly Phe Arg Cys
        195                 200                 205

Phe Gln Arg Phe Leu Arg Glu
    210                 215

<210> 6
<211> 1043
<212> PRT
<213> Arabidopsis thaliana

<400> 6

Met Met Pro Val Tyr Met Asp Pro Ser Gln Pro Cys Gln Met Arg Pro
1               5                   10                  15

Gln Glu Tyr Tyr Tyr Gln Gly Phe Gly Asn Asn Ser Gln His Met Ala
            20                  25                  30

Met Asp Ala Pro Pro Pro Cys His Gly Ser Cys Val His Gly Asn Phe
        35                  40                  45

Pro Ala Tyr Trp Pro Pro Cys Tyr Pro Pro Gln Val Pro Tyr His Gln
    50                  55                  60

Cys Cys Met Asn Arg Ser Ala Phe His Pro Pro His Ala Ser Tyr Ala
65                  70                  75                  80

Pro Ser Cys Tyr Val His Pro Pro Phe Pro Val Gly Tyr Gln Pro Trp
                85                  90                  95

Phe Asp Val Glu Lys Asp Val Pro Gly Lys His His Cys Gly Lys Cys
            100                 105                 110

Ser Ser Gln Met Cys Asp Leu Lys Lys Asp Arg Gly Val Val Ile Glu
        115                 120                 125

Glu His Glu Pro Glu Ile Glu Lys Gly Glu Ala Val Leu Pro Val Arg
    130                 135                 140

Ser Thr Asn Cys Pro Tyr Pro Ile Ile Trp Ile Pro His Glu Asn Ala
145                 150                 155                 160

Arg Asn Gln Glu Tyr Arg Ser Ser Leu Gly Leu Gly Lys His Asn Gln
                165                 170                 175

Pro Pro Ala Glu Val Arg Ala Pro Asp Asn Met Thr Ile Gln Lys Ser
            180                 185                 190

Phe Pro Glu Ser Trp Arg Gly Cys Phe Pro Phe Asp Glu Ser Ser Met
        195                 200                 205

Lys Ser Leu Val Gln Asn Gln Asp Ser Lys Lys Ala Gln Asn Gly Lys
    210                 215                 220

Thr Val Glu Ala Pro Phe Asp Ile Ser Lys Phe Lys Ser Leu Leu Gln
225                 230                 235                 240

Gly Gln Asp Met Lys Glu Ala Gln Ile Gln Lys Asn Lys Glu Glu Leu
                245                 250                 255

Gly Gln Leu Thr Tyr Pro Thr Ser Trp Val Pro Ser Arg Arg Lys Arg
            260                 265                 270

Asp Asp Val Glu Ala Ser Glu Ser Ser Asn Glu Asp Arg Lys Lys Met
        275                 280                 285

Gln Asn Gly Lys Thr Val Glu Tyr Pro Phe Asp Ile Ser Met Ile Lys
    290                 295                 300

Ser Leu Ile Gln Gly Gln Asp Val Lys Glu Ala Gln Asn Gln Lys Asn
305                 310                 315                 320

Lys Glu Glu Pro Gly Gln Val Pro Tyr Pro Ile Phe Trp Ile Pro Ser
                325                 330                 335

Tyr Gly Lys Arg Lys Asp Val Glu Ala Ser Glu Ser Lys Glu Ser Ser

            340                 345                 350

Asn Glu Gly Arg Asn Leu Glu Ser Cys Pro Ser Asp Leu His Arg Asn
        355                 360                 365

Glu Gly Gln Ile Thr Gln Ala Lys Gly Lys Glu Gly Asn Phe Glu Cys
    370                 375                 380

Asn Val Leu Ser Asp Ala Glu Glu Lys Ser Ser Val Ile Asn Ile Pro
385                 390                 395                 400

Val Ala Asn His Leu Gln Glu Pro Arg Asn Ile Pro Val Lys Leu Ser
                405                 410                 415

Glu Asn His Leu Pro Lys Pro Thr Glu Pro Thr Lys Arg Ile Ala Lys
            420                 425                 430

Asn Glu Pro Val Lys Ser Thr Lys Lys Glu Gln Ser Ser Ser Ser Ser
        435                 440                 445

Glu Ala Ser Lys Leu Pro Pro Val Cys Leu Arg Val Asp Pro Leu Pro
    450                 455                 460

Lys Glu Arg Asn Gly Gly Ser Lys Ser Val Ser His Pro Lys Arg Met
465                 470                 475                 480

Glu Lys Ser Lys Glu Thr Lys Ile Ala Ala Pro Leu Ser Ser Lys Lys
                485                 490                 495

Ala Glu Ser Arg Thr Val Pro Glu Ala Cys Asn Val Lys Cys Glu Asp
            500                 505                 510

Ala Asn Ala Glu Met Lys Met Ala Glu Gly Ser Leu Asn Ala Leu Arg
        515                 520                 525

Thr Glu Lys Gly Ser Val Glu Ser Asn Ser Asn Leu Gln Glu Glu Ser
    530                 535                 540

Asn Gly Glu Ile Ile Lys Pro Cys Glu Ala Lys Glu Asn Arg Glu Gln
545                 550                 555                 560

Pro Ala Lys Lys Ser Phe Thr Glu Glu Glu Ala Ala Arg Ile Ile Gln
                565                 570                 575

Ser Met Tyr Arg Gly Tyr Asp Val Arg Arg Trp Glu Pro Ile Lys Lys
            580                 585                 590

Leu Lys Glu Ile Ala Thr Val Arg Glu Gln Met Gly Asp Val Lys Lys
        595                 600                 605

Arg Ile Glu Ala Leu Glu Ala Ser Thr Asp Gln His Ile Glu Glu Lys
    610                 615                 620

Glu Ile Val Val Asn Gly Glu Leu Val Met Asn Leu Leu Leu Lys Leu
625                 630                 635                 640

Asp Ala Val Glu Gly Leu His Pro Ser Ile Arg Glu Phe Arg Lys Ala
                645                 650                 655

Leu Ala Thr Glu Leu Ser Ser Ile Gln Asp Lys Leu Asp Ser Leu Lys
            660                 665                 670

Asn Ser Cys Ala Ser Ala Glu Lys Glu Ala Val Lys Glu Gln Val Glu
        675                 680                 685

Ile Lys Ser Gln Pro Ser Asp Ser Pro Val Asn Leu Glu His Ser Gln
    690                 695                 700

Leu Thr Glu Glu Asn Lys Met Val Ser Asp Thr Asn Leu Glu Lys Val
705                 710                 715                 720

Leu Arg Leu Ser Pro Glu Glu His Pro Met Ser Val Leu Asn Arg Thr
                725                 730                 735

Asp Glu Lys Gln Ala Glu Ser Ala Ala Glu Thr Glu Glu Gly Tyr Gly
            740                 745                 750

Leu Phe Glu Thr Leu Ala Thr Asp Ser Lys Gln Ala Thr Glu Asn Ala
        755                 760                 765

Ala Ala Ala Ser Ser Thr Thr Ile Pro Glu Lys Ile Gly Glu Val Glu
    770                 775                 780

Thr Val Val Pro Gly Asn Pro Pro Ser Ala Asp Gly Asn Gly Met Thr
785                 790                 795                 800

Val Thr Asn Val Glu Glu Asn Lys Ala Met Val Val Glu Ser Leu Glu
                805                 810                 815

Glu Pro Ile Asn Glu Leu Pro Gln Met Val Glu Glu Thr Glu Thr Asn
            820                 825                 830

Ser Ile Arg Asp Pro Glu Asn Ala Ser Glu Val Ser Glu Ala Glu Thr
        835                 840                 845

Asn Ser Ser Glu Asn Glu Asn Arg Lys Gly Glu Asp Asp Ile Val Leu
    850                 855                 860

His Ser Glu Lys Asn Val Glu Leu Ser Glu Leu Pro Val Gly Val Ile
865                 870                 875                 880

Asp Glu Glu Thr Gln Pro Leu Ser Gln Asp Pro Ser Ser Ser Tyr Thr
                885                 890                 895

Arg Glu Gly Asn Met Thr Ala Met Asp Pro Lys Thr Ala Ser Gln Glu
            900                 905                 910

Glu Thr Glu Val Asp His Ser Pro Asn Asn Ser Lys Gly Ile Gly Gln
        915                 920                 925

Gln Thr Ser Glu Pro Gln Asp Glu Lys Glu Gln Ser Pro Glu Thr Glu
    930                 935                 940

Val Ile Val Lys Glu Gln Pro Leu Glu Thr Glu Val Ile Leu Asn Glu
945                 950                 955                 960

Gln Ala Pro Glu Pro Glu Ile Thr Glu Pro Gly Ile Ser Lys Glu Thr
                965                 970                 975

Lys Lys Leu Met Glu Glu Asn Gln Arg Phe Lys Glu Thr Met Glu Thr
            980                 985                 990

Leu Val Lys Ala Gly Arg Glu Gln  Leu Glu Val Ile Ser  Lys Leu Thr
        995                 1000                1005

Ser Arg  Val Lys Ser Leu Glu  Lys Lys Leu Ser His  Lys Lys Lys
    1010                1015                1020

Thr Gln  Ile Arg Arg Arg Ala  Ser Lys Pro Met Ser  Val Ser Pro
    1025                1030                1035

Thr Asp  Ala Val Leu
    1040

<210>  7
<211>  446
<212>  PRT
<213>  Arabidopsis thaliana

<400>  7

Met Thr Leu Phe His Arg Leu Asp Leu Ile Asp Pro Tyr Thr Cys Thr
1               5                   10                  15

Pro Leu Ile Val Arg Glu Thr Ser Ile Val Glu Pro Ser Ser Leu Phe
            20                  25                  30

Leu Gly Phe Pro Ser Phe Ile Asp Glu Asp Ile Glu Asp Leu Phe Glu
        35                  40                  45

Phe Ser Ser Pro Asn Pro Leu Asp Leu Phe Glu Thr Val Thr Asp Leu
    50                  55                  60

Val Lys Ile Lys Lys Ser Pro Ser Ser Cys Lys Tyr Lys Val Ile Arg
65                  70                  75                  80

Arg Arg Leu Glu Pro Glu Tyr Pro Leu Lys Tyr Leu Cys Asp Arg Val
                85                  90                  95

Ser Asp Leu Glu Ser Lys Phe Asp Arg Leu Val Ser Pro Lys Ser Asp
            100                 105                 110

Arg Lys Tyr Thr Leu Thr Lys Glu Ile Lys Gly Ser Gly Glu Arg Lys
        115                 120                 125

Tyr Lys Trp Glu Ala Glu Ile Gln Gly Pro Leu Glu Arg Lys Tyr Lys
    130                 135                 140

Leu Glu Ala Glu Ile Glu Gly Ser Gly Glu Arg Lys Tyr Arg Trp Thr
145                 150                 155                 160

Thr Glu Ile Lys Gly Gly Lys Lys Asp Glu Glu Gly Leu Lys Leu Ala
                165                 170                 175

Ala Leu Lys Lys Glu Lys Ala Lys Ala Lys Ala Ile Ala Ala Ala Glu
            180                 185                 190

Ala Glu Lys Lys Lys Asn Lys Asn Lys Lys Lys Ser Tyr Asn Trp Thr
        195                 200                 205

Thr Glu Val Lys Ser Glu Arg Glu Asn Gly Glu Val Ser His Thr Tyr
    210                 215                 220

Ile Ile Lys Ala Thr Thr Gly Gly Glu Lys Lys Lys Lys His Glu Glu
225                 230                 235                 240

Lys Glu Lys Lys Glu Lys Ile Glu Thr Lys Ser Lys Lys Lys Glu Lys
                245                 250                 255

Thr Arg Val Val Val Ile Glu Glu Glu Glu Glu Glu Asp Asp Glu Ser
            260                 265                 270

Ser Glu His Gly Ala Ile Val Leu Arg Lys Ala Phe Ser Arg Arg Asn
        275                 280                 285

Gly Ala Val Arg Thr Lys Lys Gly Lys Asn Lys Glu Met Pro Pro Glu
    290                 295                 300

Tyr Ala Ala Val Met Ile Gln Arg Ala Phe Lys Ala Tyr Leu Ile Arg
305                 310                 315                 320

Arg Ser Lys Ser Leu Arg Ala Leu Arg Asp Leu Ala Ile Ala Lys Thr
                325                 330                 335

Lys Leu Lys Glu Leu Arg Ala Ser Phe His Asn Phe Ser Tyr Arg Arg
            340                 345                 350

Leu Ile Ala Arg Asp Gly Glu Glu Arg Gln Lys Phe Ser Glu Lys Ile
        355                 360                 365

Ile Val Leu Leu Leu Thr Val Asp Ala Ile Glu Gly Val Asp Val Met
    370                 375                 380

Val Arg Gly Ala Lys Arg Ser Met Val Asp Glu Leu Glu Ala Met Leu
385                 390                 395                 400

Asp Val Val Asp Pro Gln Pro Gln Gly Lys Ser Leu Ser Met Arg Arg
                405                 410                 415

Arg Thr Phe Asp Met Pro Asp Ser Leu Ile Arg Lys Glu Ile Ala Glu
            420                 425                 430

Gly Val Thr Gln Ile Val Gln Met Leu Glu Thr Glu Glu Glu
        435                 440                 445

<210>  8
<211>  262
<212>  PRT
<213>  Oryza sativa

<400>  8

Met Met Ser Gly Val Gly Gly Gly Arg Ser Gly Gly Arg Asp Ala Glu
1               5                   10                  15

Gly Glu Trp Glu Val Arg Pro Gly Gly Met Leu Val Gln Arg Arg Asp
            20                  25                  30

Gly Asp Thr Gly Pro Ala Val Arg Leu Arg Val Ser His Gly Ala Ser
        35                  40                  45

Phe Arg Asp Val Ala Val Pro Ala His Ser Thr Phe Gly Glu Leu Lys
    50                  55                  60

Gly Val Leu Thr Gln Ala Thr Gly Val Glu Pro Glu Arg Gln Arg Leu
65                  70                  75                  80

Phe Phe Arg Gly Lys Glu Lys Ser Asp Asn Glu Phe Leu His Thr Ala
                85                  90                  95

Gly Val Lys Asp Gly Ala Lys Leu Leu Leu Leu Glu Lys Pro Ala Pro
            100                 105                 110

Ala Asn Val Glu Gln Arg Ala Glu Pro Val Ile Met Asp Glu Ser Met
        115                 120                 125

Met Lys Ala Cys Glu Ala Val Gly Arg Val Arg Ala Glu Val Asp Arg
    130                 135                 140

Leu Ser Ala Lys Val Cys Asp Leu Glu Lys Ser Val Phe Ala Gly Arg
145                 150                 155                 160

Lys Ile Glu Asp Lys Asp Phe Val Val Leu Thr Glu Leu Leu Met Met
                165                 170                 175

Glu Leu Leu Lys Leu Asp Gly Ile Glu Ala Glu Gly Glu Ala Arg Ala
            180                 185                 190

Gln Arg Lys Ala Glu Val Arg Arg Val Gln Gly Leu Val Glu Thr Leu
        195                 200                 205

Asp Lys Leu Lys Ala Arg Asn Ala Asn Pro Phe Ser Asp Gln Asn Lys
    210                 215                 220

Ser Val Ser Val Thr Thr Gln Trp Glu Thr Phe Asp Asn Gly Met Gly
225                 230                 235                 240

Ser Leu Asn Ala Pro Pro Pro Arg Val Ser Ser Thr Gln Ile Asn Thr
                245                 250                 255

Asp Trp Glu Gln Phe Asp
            260

- 15 -

15

1